(19) Europäisches Patentamt
European Patent Office
Office européen des brevets

(11) **EP 4 312 928 B1**

(12) **EUROPEAN PATENT SPECIFICATION**

(45) Date of publication and mention
of the grant of the patent:
**24.06.2026 Bulletin 2026/26**

(21) Application number: **22715290.7**

(22) Date of filing: **21.03.2022**

(51) International Patent Classification (IPC):
*A61F 13/505* (2006.01)   *A61F 13/539* (2006.01)
*A61F 13/56* (2006.01)   *A61F 13/70* (2006.01)
*A61F 13/74* (2006.01)   *A61F 13/80* (2006.01)

(52) Cooperative Patent Classification (CPC):
**A61F 13/505; A61F 13/539; A61F 13/5633;
A61F 13/70; A61F 13/74; A61F 13/80**

(86) International application number:
**PCT/US2022/021086**

(87) International publication number:
**WO 2022/203989 (29.09.2022 Gazette 2022/39)**

(54) **MULTI-PIECE ABSORBENT ARTICLE**

MEHRTEILIGER SAUGFÄHIGER ARTIKEL

ARTICLE ABSORBANT EN PLUSIEURS PARTIES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.03.2021   US 202163164687 P
03.11.2021   US 202163275104 P**

(43) Date of publication of application:
**07.02.2024 Bulletin 2024/06**

(73) Proprietor: **The Procter & Gamble Company
Cincinnati, OH 45202 (US)**

(72) Inventors:
• **ARIZTI, Blanca
Cincinnati, Ohio 45202 (US)**
• **SCHOENBORN, Udo Friedel
65824 Schwalbach Am Taunus (DE)**
• **ISHIHARA, Kaoru
Cincinnati, Ohio 45202 (US)**
• **MORAND, Matthias
65824 Schwalbach Am Taunus (DE)**

(74) Representative: **P&G Patent Germany
Procter & Gamble Service GmbH
Sulzbacher Straße 40
65824 Schwalbach am Taunus (DE)**

(56) References cited:
**WO-A1-2014/138274      US-A1- 2008 004 584**

**Description**

FIELD

The present invention relates generally to the field of wearable absorbent articles having absorbent inserts and reusable outer covers.

BACKGROUND

Absorbent articles (*e.g.,* diapers, adult incontinence articles, feminine hygiene pads) offer the benefit of receiving and containing urine and/or other bodily exudates (*e.g.,* feces, menses, mixture of feces and urine, mixture of menses and urine, etc.). It has been proposed to manufacture two-piece absorbent articles with a reusable outer cover and a detachable absorbent insert that may be reusable or disposable. In this way, the insert can be made with different materials to enhance performance and less energy may be consumed as the insert can be separately laundered. Further, where disposable, the inserts may be made with materials known to provide even more superior performance, while minimizing the amount of waste as the whole article need not be disposed.

Despite several designs of a two-piece absorbent article, the designs still present some disadvantages. Indeed, many configurations have insufficient attachment between the insert and outer cover, causing discomfort and/or leakage. Other configurations may provide strong adhesion between the components, but such attachment is difficult to release, causing the caregiver or wearer to struggle with or even rip the soiled insert upon removal. And in some instances, absorbent particulate, exudates, or other materials that were contained within the article may be exposed. Further, when attachment is too strong, remnants of the insert may be left on the outer cover, or vice versa, after detachment.

WO 2014/138274 A1 relates to pants that include reusable outer covers and disposable absorbent inserts. The outer cover comprises a fastening zone positioned in the front waist region and the rear waist region and has a certain average modulus.

Therefore, there is a need for a two-piece absorbent article that provides sufficient attachment between the components while permitting easy detachment.

SUMMARY

The invention provides an absorbent article as defined in the claims.

BRIEF DESCRIPTION OF THE DRAWINGS

Fig. 1 is a perspective view of a wearable absorbent article as it might appear being worn by a wearer about the lower torso;
Fig. 2 is a plan view of a wearable absorbent article opened and laid flat, inner surface facing the viewer;
Fig. 3 is an exploded cross sectional view of the exemplary absorbent article in Fig. 2, taken along line 3-3;
Fig. 4 is an outer cover opened and laid flat, inner surface facing the viewer;
Fig. 5 is a plan view of an insert opened and laid flat, outer (garment-facing) surface facing the viewer;
Fig. 6 is a perspective view of a disposable absorbent insert shown apart from an outer cover, as it might appear in a free-standing, relaxed state;
Fig. 7 is a plan view of a disposable absorbent insert shown stretched out and laid flat, wearer-facing surfaces facing the viewer;
Fig 8 is a schematic, side elevation view of a specimen for use in the Peel Strength Test Method described herein;
Fig. 9 is a schematic side view of an absorbent insert bonded to a release paper in an engagement region; and
Fig. 10 is a schematic side view of an absorbent insert bonded to a release paper in an engagement region.

DETAILED DESCRIPTION

"Absorbent article" means a device that absorbs and contains body exudates and, more specifically, devices that are placed against or in proximity to the body of the wearer to absorb and contain the various exudates discharged from the body. Exemplary absorbent articles include diapers, training pants, pull-on pant-type diapers (i.e., a diaper having a pre-formed waist opening and leg openings such as illustrated in U.S. Patent No. 6,120,487), refastenable diapers or pant-type diapers, incontinence briefs and undergarments, diaper holders and liners, feminine hygiene garments such as panty liners, absorbent inserts, and the like.

"Absorbent insert" and "insert" mean a component of a wearable absorbent article that is adapted to contain and/or absorb urine, feces, menses, or any combination thereof, and is adapted to be installable and removable as a modular unit, from an outer cover. Herein, an absorbent insert may also be referred to as an "absorbent assembly". The terms "absorbent insert," "insert" and "absorbent assembly" may be used interchangeably herein.

"Outer cover" means a component of a wearable absorbent article that is adapted to be worn about the lower torso of a wearer, and is adapted to support or in normal use is capable of supporting and holding an absorbent insert. The term encompasses a wrapping structure (such as included in a conventional diaper) and a pant structure (such as included in underwear for the lower torso, of any design).

"Disposed" refers to an element being located in a particular place or position. A feature that is disposed on a surface or side of a component may be integral with said component or may be joined to said component.

"Disposable," when referring to an absorbent insert, means that the absorbent insert is not adapted or intended to be effectively sanitarily laundered in an ordinary household laundering process and ordinary household equipment, and thereby is ordinarily unsuitable for sanitary and effective reuse so as to provide as-new intended functions and performance, following soiling by exudates and removal from an outer cover. By way of non-limiting examples, effective laundering may be frustrated or prevented, causing the insert to be disposable, by inclusion of materials and/or construction: that do not retain their substantial as-new physical shape or structure through ordinary household laundering and drying so as to be effective as-new in reuse; that absorb aqueous liquids and cannot be sufficiently dried/dehydrated in ordinary household drying equipment and ordinary drying cycles so as to be effective as-new in reuse; that dissolve or substantially degrade in ordinary household laundering or drying, causing the insert to be substantially damaged or rendered useless; and/or that cannot be effectively cleaned of exudate material through ordinary laundering, so as to be sanitary and otherwise acceptable for re-use.

Overview

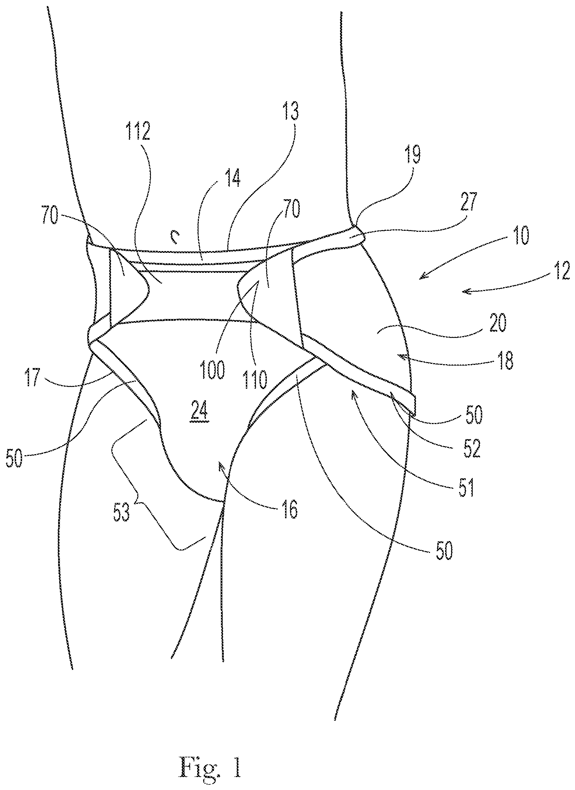

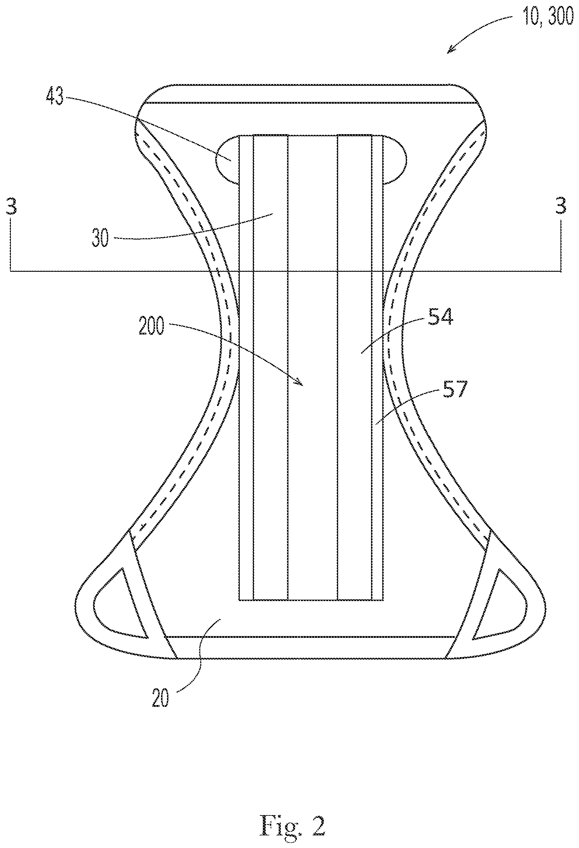

Fig. 1 is a perspective view an exemplary, nonlimiting embodiment of an absorbent article 10. The article is shown in the form of a two-piece absorbent article 12, having an outer cover 20 and an absorbent insert 30 shown in Fig. 2. It is to be understood that during manufacturing, the article may comprise several discrete pieces that are joined together. However, by two-piece absorbent article, it is meant that the article in its final form has two components that the user assembles together for wear. While shown as a two-piece absorbent article, it is also contemplated that the article may comprise more than two pieces, such as attachable ears or belts. Returning to Fig. 1, it can be seen that the absorbent article 10 may have a front region 14, a rear region 18 and a crotch region 16 disposed between the front and rear regions. The article may be placed on a wearer by wrapping the outer cover 20 between the wearer's legs and under the buttocks such that the crotch region 16 is between the wearer's legs. When the insert 30 has been installed into outer cover 20, the insert 30 will then be disposed within outer cover 20, next to the wearer. Nonlimiting examples of two-piece articles are disclosed in U.S. Pat. Nos. 8,998,870, 9,089,456, 8,435,223, 9,011,402, 8,808,263, 8,759,605 and 8,932,273 and 9,078,789.

As shown in Figs. 2-3, the absorbent insert is engageable with the outer cover in an engagement region 200 using a fastening system 100. When engaged, the article comprises an insert-outer cover peel strength measured in N/m in the engagement region. The absorbent insert 30 comprises a backsheet 34 having an inner layer 85 and an exterior layer 86. The inner layer may comprise a film and the exterior layer may comprise a nonwoven material. The inner and exterior layers are joined in the engagement region 200 and comprise a backsheet layer peel strength measured in N/cm. The backsheet layer peel strength is greater than the insert-outer cover peel strength.

Further, the absorbent insert may comprise an absorbent core 44 having absorbent material 45 disposed on and/or between substrates 49. The backsheet 34 may be joined to at least one substrate 49 in the engagement region, and the article comprises a backsheet-to-core substrate peel strength measured in N/cm. The backsheet-to-core substrate peel strength is greater than the insert-outer cover peel strength. These and additional features are discussed in more detail below.

Two-Piece Article

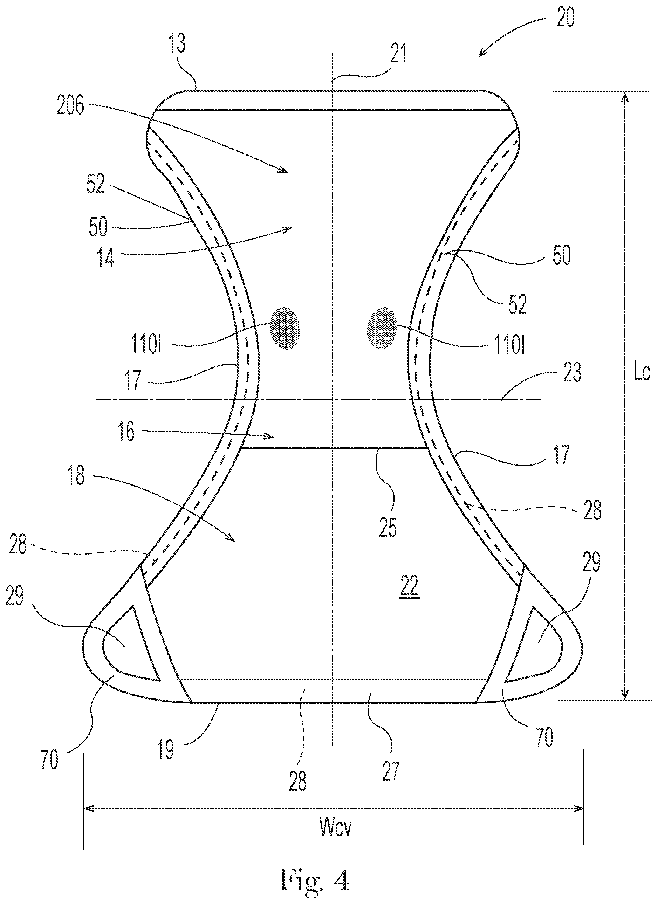

An absorbent article in accordance with present disclosure comprises an outer cover and an absorbent insert. In various embodiments, the absorbent insert is disposable, and the outer cover is reusable. Turning to Fig. 4, an exemplary outer cover 20 is shown in a flat configuration with the wearer- facing surface 22 facing the viewer. The outer cover comprises a front lateral edge 13, a rear lateral edge 19, and two longitudinal edges 17. The outer cover comprises a length, Lc, from the outboard most portion of the front lateral edge to the outboard-most portion of the rear lateral edge. The outer cover may comprise a lateral axis 23, dividing evenly its length, and a longitudinal axis 21. The longitudinal edges 17 may be parallel to the longitudinal axis 21. However, for better fit, longitudinal edges 17 may be curved or angled to produce, for example, an "hourglass" shape article when viewed in a plan view as shown in Fig. 2, for example. In nonlimiting examples, the outer cover may be asymmetric, for instance having a width in its rear region that is greater than the width in its front region. Thus, the width of the outer cover, Wcv, as measured between the longitudinal edges along a line parallel to the lateral axis, may vary along the longitudinal length of the outer cover. Nonlimiting examples of outer covers are disclosed in U.S. Pat. Nos. 9,387,138 and 8,435,223.

Outer cover 20 and/or layers or portions thereof may be made of any durable or semi-durable knitted, woven, or nonwoven textile or textile-like material that is appropriately compatible with skin of the intended wearer(s). Suitable examples are described in U.S. Applications Ser. Nos. 12/687,493; 12/687,412; 12/687,528; and 12/687,425 (all by Roe et al.).

Non-limiting examples of fibers, nonwovens and laminates of nonwovens and films that might be considered for use as semi-durable outer cover materials may be found in U.S. Patents Nos. 7,223,818; 7,211,531; 7,060,149; 6,964,720; 6,905,987; 6,890,872; 6,884,494; 6,878,647; and 5,518,801; and U.S. Published Applications Nos. 2008/0319407; 2008/0045917; 2007/0293111; 2007/0287983; 2007/0287348; 2007/0249254; 2007/0203301; and 2005/0164587.

The outer cover 20 may be formed of a single layer of a durable or semi-durable material or may be formed from two or more layers, which may be joined together at one or more seams 25. In nonlimiting examples, a garment-facing surface 24 is formed form a different material than a wearer-facing surface. For example, materials forming the garment-facing surface of the outer cover may comprise greater hydrophobicity than materials forming the wearer-facing surface. Likewise, different regions of the same surface may be formed by different materials. For example, the material predominately forming the inner surface of rear region 18 may be selected primarily for its elasticity features, which may better serve to provide snug fit about wearer body contours and accommodate wearer movement (i.e., about the buttocks and hips). By comparison, the material predominately forming the inner surface of front region 14 and/or crotch region 16 might be selected primarily for its hydrophobicity or hydrophilicity, which may better serve to contain liquid exudates.

Additionally, in some circumstances, it may be desirable that the material(s) selected for inner surfaces 22 have soft tactile properties so as to have a pleasant feel against the skin, particularly in areas where no portion of an insert is expected to be present between the outer cover and the wearer's skin. Further, it may be desirable that at least a portion of the inner surface comprise a material that is engageable by fastening components. Additionally, or alternatively, a second layer of material may be formed of a textile material having enhanced elasticity, such as by inclusion of fibers of an elastomeric material (such as spandex). In another example, an intermediate film layer may be included, laminated or not laminated with another layer.

Layers or other elements of the outer cover may be joined to each other via any suitable mechanism, including, for example, adhesives, mechanical bonding, ultrasonic bonding, sewing, stitching, serging, edging, and the like.

Waist Features, Leg Gasketing Systems, Ears

Still referring to Fig. 4, the article and outer cover may comprise one or more waist features 27. The waist feature may be elastic and thereby provide better fit about the waist of the wearer. Elasticized waist features include waistbands, waist cuffs having pockets formed from a portion of the waist feature that is unattached from the remainder of the outer cover, and waist panels and/or belts designed to fit securely about the abdomen of the wearer. Nonlimiting examples of elasticized waist features are disclosed in U.S. Patent App. Nos. 14/533,472; 15/074,675 and 62/855,001. Elasticized waist features may comprise one or more nonwoven or textile layers, which may be layers of the outer cover or discrete portions, and one or more elastic elements 28. In nonlimiting examples, the elasticized waist feature comprises elastic strands joined to the nonwoven and/or textile layer(s). In further nonlimiting examples, the elasticized waist feature comprises a laminate of one or more textile and/or nonwoven layers and one or more films. The elasticized waist element may comprise one or more rugosities if the elastic material is strained prior to lamination. In other nonlimiting examples, the layers of the elastic laminate may be joined at zero applied strain and subsequently activated.

In alternative embodiments, the waist feature may be inelastic. In such configurations, the waist feature may provide additional anchoring about the waist of the wearer.

Additionally, or alternatively, the article may comprise a leg gasketing system 50, portions of which may be formed by the outer cover and/or the absorbent insert. The outer cover may include one or more elastic elements 28, such as films or elastic strands, extending through all or a portion of the leg opening 51 to form a leg band portion 52. The elastic elements may be laminated with one or more nonwoven layers and/or one or more textile layers. As described with respect to the waist feature, the leg band portion may include rugosities as result of the lamination process. In other nonlimiting examples, the leg band portion may be a zero strain, activated laminate.

The waist features and/or leg band portions may be disposed along the edge of the outer cover, and in some circumstances, it may be desired to have elasticized waist and leg band portions situated along substantially the entire length of the leg and/or waist openings so as substantially or completely encircle the wearer's legs and/or waist while outer cover 20 is worn. The gathered material within rugosities can serve to accommodate stretching of waist feature and leg band portions. This arrangement not only may provide for better fit about the wearer's legs, but also may enable the outer cover 20, when formed of appropriately sized and shaped material, to form a pouch-like structure 53 in the crotch region (see Fig. 1) when worn, which may serve to provide space within the outer cover to accommodate the insert 30 and help hold it in place within outer cover 20, in a substantially laterally centered position within the crotch region. The outer cover 20 may also include anchoring supplements, bands or systems thereof as described in more detail in U.S. Patent No. 8,932,273.

The outer cover may include ears 70 in one or both of the front and back regions. As shown in Fig. 1, the ears may include fastening components 110 such that the ears can be secured to the opposing region (e.g., rear ears may include fastening components that may engage with the front region). The outer cover may include receiving components 112 to operatively engage with fastening components 110. Nonlimiting examples of engageable fastening and receiving components include tape tabs, hook and loop fastening components, interlocking fasteners such as tabs & slots, buckles, buttons, snaps, and/or hermaphroditic fastening components. Some exemplary surface fastening systems are disclosed in U.S. Patent Nos. 3,848,594; 4,662,875; 4,846,815; 4,894,060; 4,946,527; 5,151,092; and 5,221,274. An exemplary interlocking fastening system is disclosed in U.S. Patent No. 6,432,098. Adhesive is also a suitable fastening component. A fastening component may be discrete from and joined to the outer cover or may be integral with the outer cover. The receiving component may be discrete from and joined to the outer cover or may be integral with the outer cover. The fastening component and/or the receiving component may further include a release tape or other material, including folded material, that protects the component from insult prior to use. In nonlimiting examples, a fastening component 110 is disposed on a wearer-facing surface 22 of the outer cover and a receiving component 112 is disposed on a garment-facing surface 24. In such nonlimiting examples, the outer cover may comprise two or more fastening systems having distinct fastening locations, which deconcentrates lateral tensile focuses and reduces the tendency of the front portion of the article to pivot around the single fastening location. Suitable dual fastening systems are disclosed for example in U.S. Pat. App. Nos. 16/684,230 and 16/545,425.

The outer cover may also comprise an insert fastening system having one or more insert fastening components 110I capable of operatively engaging with an insert receiving component 112I disposed on the absorbent insert, as shown in Figs. 4-5 for example. The insert fastening and receiving components may comprise any of the exemplary components disclosed.

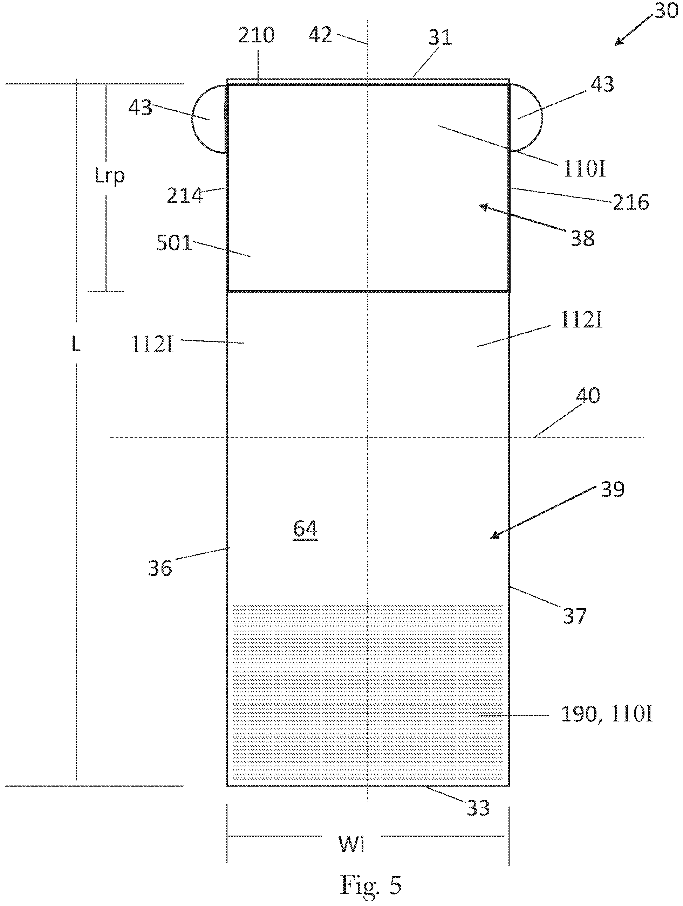

The fastening and/or receiving components may be discrete from and joined to the outer cover or absorbent insert or may be integral with one or both. In nonlimiting examples, fibrous material (such as nonwoven material forming portions of the outer cover surfaces or portions of the insert backsheet or topsheet) may be comprise integral loops material as illustrated in Fig. 5. Further to the above, it is to be understood fastening systems need not necessarily include respective components of a two-component fastening system. Rather, a fastening system may require only one component. By way of nonlimiting example, a fastener component on outer cover 20 may include a patch of adhesive; a structure having a region of relatively high coefficient of friction; a pocket 29; flap; strap; or other capturing, holding and/or retaining surface, device, or structure. Thus, a receiving component is unnecessary. Referring to Fig. 4, in some nonlimiting examples, the outer cover 20 may include one or more pocket structures 29 situated on or along the inner surface 22. A pocket structure may be adapted to receive, fit, and capture, for example, the forward edge and a portion of forward region 38 of insert 30.

Absorbent Insert

Returning to Fig. 5, the absorbent insert 30 may be designed to contain and/or absorb body exudates, and may be made of pliable materials as will be described further below. The insert 30 includes a forward region 38 and a rearward region 39, a first lateral end 31 and a second lateral end 33, a first longitudinal edge 36, a second longitudinal edge 37, a lateral axis 40 and a longitudinal axis 42. The insert 30 comprises a length L from the outboard-most portion of front edge 31 to the outboard-most portion of the rear edge 33. The lateral axis equally divides the length L. In nonlimiting examples, the insert length L is less than the length of the outer cover Lc.

The insert may comprise one or more insert fastening components 110I, such as one or more areas of adhesive 190. Additionally, or alternatively, the insert may comprise one or more insert receiving components 112I. The insert fastening components may be disposed proximate to one or more lateral ends of the absorbent insert. Additionally, or alternatively, the insert fastening and/or receiving components may overlap, or have a portion within 1 mm of, the lateral axis 40. The insert fastening and/or receiving components may overlap, or have a portion within 1 mm of, a line parallel to the lateral axis and disposed a longitudinal distance equal to L/4 from the first end 31. Additionally, or alternatively, the insert fastening and/or receiving components may at least part may overlap, or have a portion within 1 mm of, a line parallel to the lateral axis and disposed a longitudinal distance equal to 0.75L from the first end 31.

The longitudinal edges 36, 37 may be generally parallel to the longitudinal axis. Alternatively, the longitudinal edges 36, 37 may be curved, such as in an hour-glass configuration. Thus, the width of the insert, Wi, may vary. In nonlimiting examples, the width of the insert, Wi, is less than the width of the outer cover, Wcv, at one or more longitudinal positions. The width of the insert, Wi, may be less than the width of the outer cover, Wcv, throughout the length of the insert.

In various embodiments, the absorbent insert may comprise a surface area of at least about 400 cm², or at least about 450 cm², or at least about 475 cm², or from about 300 cm² to about 600 cm², or from about 400 cm² to about 500 cm², reciting for each range every 10 cm² increment therein, on its wearer-facing surface and/or on its garment-facing surface. In this way, the absorbent insert may accommodate relatively large wearers (toddlers) while accommodating smaller sized wearers (infants). In some embodiments, the absorbent insert may be foldable, further allowing the size to be adjusted for different wearers, as is disclosed in commonly assigned U.S. Pat. App. No. 63/028021. The absorbent insert may comprise a caliper of about 20 mm or less, or about 15 mm or less, or from about 15 mm to about 30 mm, according to the Caliper Test Method herein.

The insert also may include one or more grasp structures 43, such as lateral side grasp structures extending from a longitudinal side as shown in Fig. 2 or grasp structures extending from a lateral end 31, 33. The grasp structures may be provided to enable the user to quickly and easily grasp the insert, handle the insert during application, and/or properly place the insert. In certain embodiments, the grasp structure may have a different tactile feel than surrounding or adjacent areas to distinguish the area and ease the user's identification of the grasp structures.

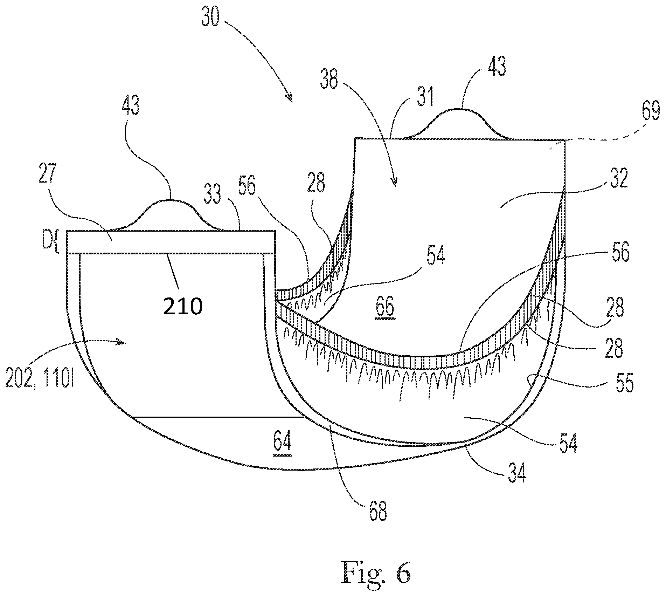

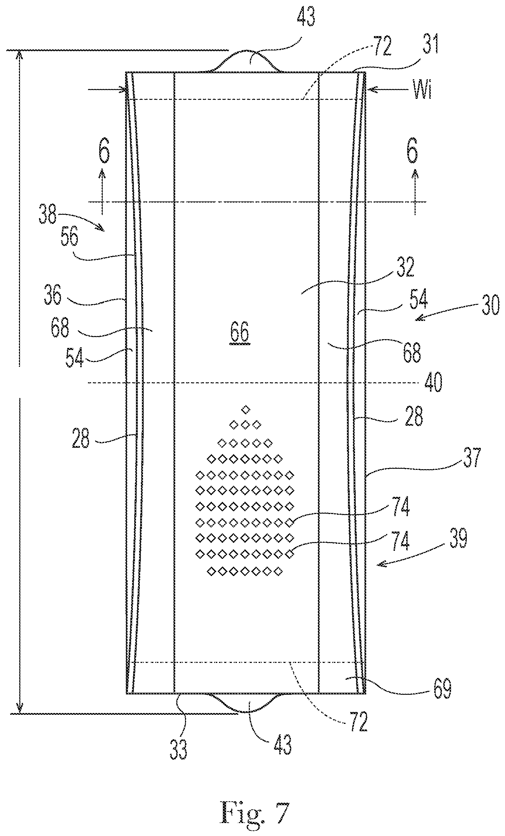

Fig. 6 depicts a disposable absorbent insert 30 in perspective view as it might appear in a free-standing, relaxed state, with both the body-facing surfaces 66 and garment-facing surfaces 64 shown. Fig. 7 depicts an example of an insert 30 shown stretched out and laid flat (against elastic-induced contraction), body-facing surfaces 66 facing the viewer.

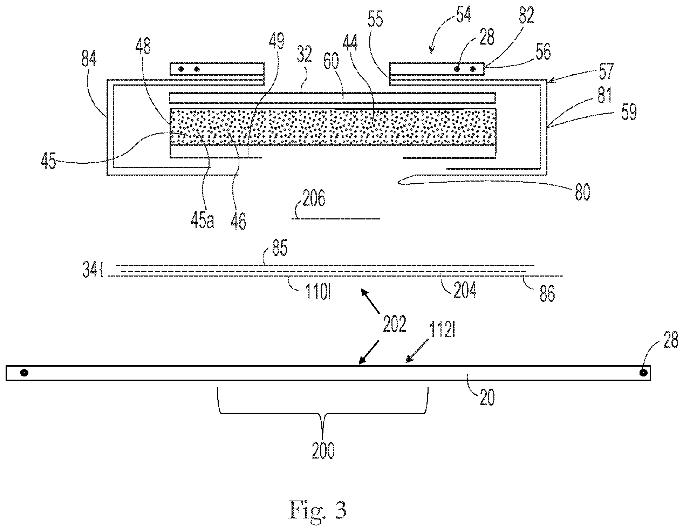

As shown in Fig. 6 for example, the insert 30 may have a topsheet 32 and a backsheet 34. The topsheet and backsheet may be joined together along longitudinal seams 68 and along lateral seams 69. An absorbent core 44 may be disposed between the topsheet and the backsheet as shown for example in Fig. 3.

Returning to Figs. 6-7, the insert 30 may further include longitudinal standing cuffs 54 affixed along the longitudinal sides 36, 37. Nonlimiting examples of absorbent inserts and details of their features are disclosed in U.S. Pat. Nos. 8,546,641 and 9,011,402. Stiffening elements 72 may also be included to aid the user in engaging the insert with the outer cover and/or help the insert maintain its intended shape and configuration during wear.

It will be appreciated that the outer cover described above can be constructed of materials and construction so as to bear and sustain a majority of the structural loading generally imposed upon a disposable diaper, by stretching and accommodation of the wearer's anatomical features and body movements, and by absorption, swelling and added weight resulting from the wearer's exudations of waste. Thus, lesser requirements for structural strength of an insert might be present with use of such an outer cover, as compared with strength required of inside components of a disposable diaper. Therefore, an article such as described herein may include a disposable absorbent insert manufactured from materials that are different from those ordinarily used in the manufacture of disposable diapers, such as petroleum-derived materials, e.g., polyethylene and polypropylene. For example, a disposable absorbent insert having one or more of a topsheet, backsheet, standing cuffs and/or other components formed of products of wood, cotton, flax (linen), hemp, bamboo, or other cellulose fibers (e.g., paper), in addition to the materials identified above, is contemplated. If resistance to aqueous liquid penetration or substantial liquid impermeability is desired, e.g., for a backsheet and/or standing cuffs, a material formed of ordinarily hydrophilic fibers such as paper may be coated or impregnated with a hydrophobic material, such as a skin-compatible oil or wax, to impart the desired resistance to aqueous liquid penetration. Each of the materials forming the insert may be selected so as to be dispersible in water or an aqueous solution, flushable, biodegradable and/or compostable (preferably to an agriculturally usable humus or soil amendment).

Topsheet

The topsheet 32 is generally a portion of the absorbent article 10 that may be positioned at least in partial contact or close proximity to a wearer. Suitable topsheets 32 are generally supple, soft feeling, and non-irritating to a wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester, or polypropylene or bicomponent PE/PP fibers or mixtures thereof), or a combination of natural and synthetic fibers. The topsheet may have one or more layers. The topsheet may be apertured, may have any suitable three-dimensional features, and/or may have a plurality of embossments (e.g., a bond pattern). The topsheet 32 may comprise one or more apertures 74. The topsheet may be apertured by overbonding a material and then rupturing the overbonds through ring rolling, such as disclosed in U.S. Patent No. 5,628,097, to Benson et al., issued on May 13, 1997, and disclosed in U.S. Pat. Appl. Publication No. US 2016/0136014 to Arora et al. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

Topsheet 32, backsheet 34 or any portion of the topsheet or backsheet may be embossed and/or matte finished to provide a more cloth-like appearance.

Backsheet

Backsheet 34 is generally the outer liner portion of insert 30 forming the garment-facing surface 64 thereof, and prevents the exudates absorbed and contained within insert 30 from wicking through and soiling the outer cover.

The backsheet 34 may comprise one or more nonwovens, elastomeric films, foams, strands, or combinations of these or other suitable materials with nonwovens or synthetic films. In various embodiments, the backsheet is a laminate of two or more layers as shown in Fig. 3. The laminate may include an inner layer 85 and an external layer 86. The exterior layer 86 may be positioned between the inner layer and the absorbent core. The exterior layer 86 is bonded to the inner layer 85 by any suitable means including but not limited to adhesive, thermal bonding, pressure bonding and combinations thereof. The bonding may be continuous or discontinuous. In embodiments where the bonding comprises adhesive bonding may be provided in patterns like slots, spirals, or the like. In various embodiment, the backsheet is a laminate of an elastomeric material, such as a film 85, and a nonwoven 86. The inner layer comprises the film, and the exterior layer comprises the nonwoven 86 in such embodiments. The inner and exterior layers may be joined by any suitable means, including but not limited to adhesive, thermal bonding, pressure bonding and combinations thereof.

The basis weight of the backsheet may be less than 70 g/m$^2$, or may be from 25 g/m$^2$ to 70 g/m$^2$, or from 25 g/m$^2$ to 60 g/m$^2$ or from 25 g/m$^2$ to 50 g/m$^2$. The inner layer (e.g., film) may have a basis weight of less than 25 g/m$^2$, or from 10 g/m$^2$ to 25 g/m$^2$, or from 10 g/m$^2$ to 20 g/m$^2$. Nonwoven webs included in the backsheet may have a basis weight of less than 40 g/m$^2$, or from 10 g/m$^2$ to 30 g/m$^2$, or from 10 g/m$^2$ to 25 g/m$^2$ (in embodiments having more than one nonwoven web, these values represent the sum for all nonwoven webs taken together).

In certain embodiments, the backsheet 34 is substantially water impermeable. The backsheet may, for example, be or comprise a thin plastic film, such as a thermoplastic film having a thickness of about 0.012 mm to about 0.051 mm. Other suitable backsheet 34 materials may include breathable materials that permit vapors to escape from the absorbent article 10 while still preventing exudates from passing through the backsheet 34.

In certain embodiments, the backsheet 34 may have a water vapor transmission rate (WVTR) of greater than about 2000 g/24h/m$^2$, greater than about 3000 g/24h/m$^2$, greater than about 5000 g/24h/m$^2$, greater than about 6000 g/24h/m$^2$, greater than about 7000 g/24h/m$^2$, greater than about 8000 g/24h/m$^2$, greater than about 9000 g/24h/m$^2$, greater than about 10000 g/24h/m$^2$, greater than about 11000 g/24h/m$^2$, greater than about 12000 g/24h/m$^2$, greater than about 15000 g/24h/m$^2$, measured according to WSP 70.5 (08) at 37.8 °C and 60% Relative Humidity. A higher WVTR may be desired in this particular application, since the insert backsheet 34 will not form the outer surface of the wearable article, as a conventional disposable diaper backsheet would, but rather, will be covered by the one or more layers of the outer cover material(s) - which themselves may act in some circumstances to reduce WVTR of the composite structure.

Other suitable materials and/or manufacturing techniques may be used to provide a suitable backsheet 34 including, but not limited to, surface treatments, particular film selections and processing, particular filament selections and processing.

Backsheet 34 may be joined to topsheet 32, absorbent core 44 or any other element of insert 30 by any suitable attachment mechanism known in the art.

Absorbent Core

Still referring to Fig. 3, the insert 30 may have an absorbent core 44 disposed within the envelope-like structure formed by the topsheet 32 and backsheet 34. The absorbent core 44 may comprise a wide variety of liquid-absorbent materials 45 commonly used in disposable diapers and other absorbent articles. Examples of suitable absorbent materials include comminuted wood pulp, which is generally referred to as air felt creped cellulose wadding; melt blown polymers, including co-form; chemically stiffened, modified, or cross-linked cellulosic fibers; tissue, including tissue wraps and tissue laminates; absorbent foams; absorbent sponges; superabsorbent polymers; absorbent gelling materials (AGM); or any other known absorbent material or combinations of materials. The amount of absorbent material, such as absorbent particulate polymer material 45a present in the absorbent core may vary, but in certain embodiments, is present in the absorbent core in an amount greater than about 80%, or greater than about 85%, or greater than about 90%, or greater than about 95% by weight of the core. In nonlimiting examples, a thermoplastic material, such as a thermoplastic adhesive composition 46, may be used to immobilize superabsorbent particles on a substrate (e.g., the topsheet, backsheet or core wrap).

Absorbent particulate polymer material 45a used in the core may have a CRC (centrifuge retention capacity) value of more than 20 g/g, or more than 22 g/g, or more than 25 g/g, for example up to 50 g/g, or up to 40 g/g, or to 30 g/g, as measured according to EDANA method WSP 241.2-05. The CRC measures the liquid absorbed by the superabsorbent polymer particles for free swelling in excess liquid. Superabsorbent polymer particles having a high CRC value may be preferred since less superabsorbent polymer particles are needed to facilitate a required overall capacity for liquid absorption.

The absorbent insert may be provided with an absorbent capacity of at least about 150 g, or at least about 200 g, or at least about 250 g, or from about 120 to about 450 g, reciting for said range every 10 increment therein. The absorbent capacity is the mathematical product of the mass of absorbent material in the insert and the CRC of said material.

In certain embodiments, at least a portion of the absorbent core is substantially cellulose free and contains less than 10% by weight cellulosic fibers, less than 5% cellulosic fibers, less than 1% cellulosic fibers, no more than an immaterial amount of cellulosic fibers or no cellulosic fibers. It should be understood that an immaterial amount of cellulosic material does not materially affect at least one of the thinness, flexibility, and absorbency of the portion of the absorbent core that is substantially cellulose free. Among other benefits, it is believed that when at least a portion of the absorbent core is substantially cellulose free, this portion of the absorbent core is significantly thinner and more flexible than a similar absorbent core that includes more than 10% by weight of cellulosic fibers.

In some embodiments, the absorbent core may comprise one or more channels, wherein said channels are substantially free of absorbent particulate polymer material. The channels may extend longitudinally or laterally. The absorbent core may further comprise two or more channels. The channels may be straight, curvilinear, angled or any workable combination thereof. In nonlimiting examples, two channels are symmetrically disposed about the longitudinal axis.

The absorbent core 44 may include a core wrap 48, comprising one or more substrates 49 to encloses the absorbent material 45. Typical substrate materials used in the production of conventional cores may be used, in particular nonwovens but also paper, tissues, films, wovens, or laminate of any of these. The core wrap may in particular be formed by a nonwoven web, such as a carded nonwoven, a spunbond nonwoven ("S") or a meltblown nonwoven ("M"), and laminates of any of these. For example, spunmelt polypropylene nonwovens are suitable, in particular those having a laminate web SMS, or SMMS, or SSMMS, structure, and having a basis weight range of about 5 gsm to 15 gsm. Suitable materials are for example disclosed in US 7,744,576, US 2011/0268932 A1, US 2011/0319848 A1 or US 2011/0250413 A1. Nonwoven materials provided from synthetic fibers may be used, such as PE, PET and in particular PP.

The core wrap may be sealed along its longitudinal edges and/or its transversal edges. In a C-wrap configuration, for example, a first substrate 49 may be placed on one side of the core and extends around the core's longitudinal edges to partially wrap the opposed bottom side of the core (see Fig. 3). The flaps of the first substrate may be glued to the second substrate to provide a strong seal. This so called C-wrap construction can provide benefits such as improved resistance to bursting in a wet loaded state compared to a sandwich seal. The front side and back side of the core wrap may then also be sealed for example by gluing the first substrate and second substrate to another to provide complete enclosing of the absorbent material across the whole of the periphery of the core.

Where channels are present, the core wrap may be bonded within one or more channels, thereby providing permanent channels which maintain their channel structure in the wet state.

The absorbent core 44 may be manufactured in a wide variety of sizes and shapes (*e.g.,* rectangular, hourglass, "T"-shaped, etc.). The configuration and construction of absorbent core 44 may also be varied (*e.g.,* the absorbent core(s) or other absorbent structure(s) may have varying caliper zones, hydrophilic gradient(s), a superabsorbent gradient(s), or lower average density and lower average basis weight acquisition zones; or may comprise one or more layers or structures). Exemplary absorbent structures for use as the absorbent core 44 are described in U.S. Patent Nos. 7,744,576; 9,072,634 and U.S. Patent App. Nos. 13/491,642 and 15/232,901.

Acquisition-Distribution System

Referring again to Fig. 3, in some embodiments, an acquisition-distribution system (ADS) 60 is disposed between the topsheet 32 and the absorbent core 44. One function of the ADS is to quickly acquire one or more of the fluids and distribute them to the absorbent core in an efficient manner. The ADS may comprise one, two or more layers, which may form a unitary layer or may remain as discrete layers which may be attached to each other. The ADS 60 may include hydrophilic materials that provide significant wicking of bodily exudates. These materials may dewater the topsheet 32 and quickly move bodily exudates into the absorbent core 44. The ADS 60 may comprise one or more nonwoven materials, foams, cellulosic materials, cross-linked cellulosic materials, air laid cellulosic nonwoven materials, spunlace materials, or combinations thereof, for example. In some instances, portions of the ADS 60 may extend through portions of the topsheet 32, portions of the topsheet 32 may extend through portions of the ADS 60, and/or the topsheet 32 may be nested with the ADS 60. Typically, an ADS 60 may have a width and length that are smaller than the width and length of the topsheet 32. The ADS may have one or more channels as described above with reference to the absorbent core 44. The channels in the ADS may align or not align with channels in the absorbent core 44. In an example, a first ADS layer may comprise an acquisition layer 62 suitable for quickly acquiring fluids, and the second layer 63 may comprise a distribution layer capable of distributing fluids. In nonlimiting examples, the acquisition layer 62 may comprise a nonwoven material and/or the distribution layer 63 may comprise a cross-linked cellulosic material. Suitable ADS are described in WO 2000/59430, WO 95/10996, U.S. Pat. No. 5,700,254, and WO 02/067809, for example.

Cuff Structures

As noted above, the article 10 may comprise a leg gasketing system 50, portions of which may be formed by the insert 30. The leg gasketing system comprises one or more cuffs. At least one cuff comprises a material edge that may be disposed below the topsheet. More particularly, the material edge may be disposed between layers, where each such layer is disposed beneath the topsheet (i.e., the material edge does not contact the topsheet). In nonlimiting examples, the at least one cuff comprises a laminate of two or more layers, and each may have a material edge disposed below the topsheet (i.e., the material edge does not contact the topsheet) as shown in Fig. 3 and discussed in more detail below.

The insert may comprise a pair of longitudinal standing cuffs 54, also referred to as barrier leg cuffs or inner cuffs. Each standing leg cuff may be formed by a piece of material which is bonded to the absorbent insert so it may extend upwards from a wearer-facing surface and provide improved containment of fluids and other body exudates approximately at the junction of the torso and legs of the wearer. The standing leg cuffs are delimited by a proximal edge 55 joined directly or indirectly to the topsheet 32 and/or the backsheet 34 and a free terminal edge 56, which is intended to contact and form a seal with the wearer's skin. In some embodiments, the free terminal edge 56 comprises a folded edge 82. The standing leg cuffs 54 extend at least partially between the front edge 31 and the rear edge 33 on opposite sides of the longitudinal centerline 42 and are at least present in the crotch region.

The standing leg cuffs may be integral with the topsheet 32 or the backsheet 34 or may be a separate material joined to the topsheet and/or backsheet. Each standing leg cuff 54 may comprise one, two or more elastic elements 28 close to the free terminal edge 56 to provide a better seal. The standing cuff may be formed of any of a variety of substrates such as plastic films and woven or nonwoven webs of natural fibers (*e.g.,* wood or cotton fibers), synthetic fibers (*e.g.,* polyester or polypropylene fibers), or a combination of natural and synthetic fibers. In certain embodiments, the standing cuffs may be formed of a substantially liquid impermeable web to contain and isolate liquid exudates from the outer cover, outer clothing, and environment of the wearer and/or may be formed of a vapor permeable web for breathability of the insert and article.

The article 10 may comprise a dual gasketing system, which includes the standing cuffs 54 and gasketing cuffs 57, also referred to as outer cuffs. The gasketing cuffs 57 may be joined to the insert 30, more particularly to the topsheet and/or backsheet. The gasketing cuffs are disposed outboard of the standing cuffs and may provide a better seal around the thighs of the wearer. The gasketing cuff 57 may comprises a material edge 80 and a free terminal edge 59. The free terminal edge 59 may comprise a folded edge 81. Each gasketing cuff may comprise one or more elastic elements 28, which may be sandwiched between other layers of material, such as the portions of material forming the attached proximal portions of the standing cuffs, topsheet, backsheet, separate gasketing cuff material, or combinations thereof. In other nonlimiting examples, the gasketing cuff is void of elastics.

The gasketing cuff may be formed of any of a variety of substrates such as plastic films and woven or nonwoven webs of natural fibers (*e.g.,* wood or cotton fibers), synthetic fibers (*e.g.,* polyester or polypropylene fibers), or a combination of natural and synthetic fibers. As noted with respect to standing cuffs 54, gasketing cuffs may likewise be formed of any suitable web materials but preferably are formed of web materials that are effectively liquid impermeable while being vapor permeable, so as to contain the wearer's liquid exudates within the insert while permitting the insert to "breathe" to avoid excess humidity within the insert (which may overhydrate the wearer's skin and promote conditions such as diaper rash). Suitable materials include nonwoven, films, elastic strands, and combinations thereof.

In further embodiments, the leg gasketing system comprises standing leg cuffs that are integral with gasketing cuffs. Indeed, the outer and inner cuff on one side of the insert may be formed from a single web of material 84. In nonlimiting examples, the inner cuff may be formed by folded the web laterally outward and the outer cuff may be formed by folding the web material laterally inward as is disclosed for example in U.S. Pat. No. 8,939,957.

At least one cuff may be folded such that its material edge is disposed below the topsheet. In the embodiment shown in Fig. 3, the gasketing cuff material edge 80 is disposed between the absorbent core 44 and the backsheet as shown in Fig. 3, or more particularly between the backsheet film and the absorbent core substrate 49. In this way, the cuff material may provide an additional barrier layer to the absorbent material while providing a finished, smooth edge for the wearer (i.e., folded edge 81). A cuff material edge may be disposed between the backsheet layers 85,86, between layers of the ADS 62, 63, between the core and the ADS, or may be attached to the exterior surface of the backsheet 64. These configurations permit the cuff material to further encapsulate portions of the absorbent insert. The material edge may be joined to the forementioned layers through any suitable means, including for example adhesive, thermal bonding, pressure bonding and combinations thereof. In various nonlimiting examples, the material edge is joined to a layer beneath the topsheet by adhesive bonding.

Masking Layers

One or more masking layers or materials may be provided in the absorbent articles 10, particularly in the absorbent insert. A masking layer may be a layer that provides a cushiony feel when the absorbent article is touched from the garment-facing surface or the wearer-facing surface. The masking layer may "mask" a grainy feel potentially caused by the absorbent material 45, such as superabsorbent polymers 45a. The masking layer may "mask" bodily exudates from being visible when viewing the wearer-facing surface or the garment-facing surface of the absorbent article 10. The masking layer may have a basis weight in the range of about 15 gsm to about 50 gsm or about 15 gsm to about 40 gsm. The masking layer may comprise one or more nonwoven materials (e.g., a hydroentangled nonwoven material), foams, pulp layers, and/or other suitable materials. The masking layer may be a portion of the cuff material; for instance, cuff material edge(s) may extend below the core, such that at least 50% of the core's width, or at least 75%, or at least 90% of the core surface is covered the cuff material. The masking layer may be the backsheet or external layer of the outer cover material. The masking layer may be the layer forming the garment-facing side or the wearer-facing side of the absorbent core 44. The masking layer may be a separate material positioned intermediate the garment-facing side of the core 44 and the liquid impermeable backsheet 34.

Engagement Region 200

The absorbent insert and outer cover are capable of being attached in an engagement region 200. The outer cover and/or absorbent insert may comprise a fastening component 110I as noted above, which may be operatively engageable with a surface of the other of the outer cover and absorbent insert and/or operatively engageable with a receiving component 112I disposed on said surface. The attachment may be formed by a first bonding 202 which may comprise any suitable means, including but not limited to adhesive, mechanical bonding, heat bonding, pressure bonding and combinations thereof. Nonlimiting examples of attachment means include adhesive patch(es), adhesive strips or tabs, hook and loop fastening components, interlocking fasteners such as tabs & slots, buckles, buttons, snaps, and/or hermaphroditic fastening components. In various embodiments, the attachment means comprises an adhesive, more particularly a pressure sensitive adhesive. The adhesive may remain tacky well below its application temperature. The adhesive may be covered with a release paper during storage and transport of the absorbent insert and/or the outer cover. For example, a suitable adhesive can be a pressure sensitive hot melt adhesive. Suitable adhesives may be disclosed in commonly assigned U.S. Patent App. No. 17/237174 under attorney docket 15780MQ. The bonding may be continuous or discontinuous. As noted above, where the bonding comprises adhesive, bonding may be provided in patterns like slots, spirals, or the like.

The rheological and mechanical behavior of the adhesive may play a role in its performance as a means of attachment. One criterion associated with the rheology of the adhesive is the damping factor (tan delta-1 and storage modulus-1). In order to accommodate the initial application, staying in place on the insert, article, and/or release paper, and removal of the absorbent insert to the outer cover of the absorbent article, the damping factor can be measured at least at two different frequency ranges. This has to do with the different time scales involved in the different phases of the adhesive's life. During application and successive initial wear, the adhesive may have a very long time to be able to flow and create intimate contact with the fibers of the outer cover, promoting bond strength. This time scale is usually in the order of 1s to 100s or higher for which it then follows that for the initial placement and stay in place criteria, the damping factor (tan delta-1 and storage modulus-1) can be between about 0.01Hz (=1/100s) to about 1Hz = (1/1s). As noted, the tan delta-1 and storage modulus-1 measurements can be important for the initial application as well as the stay in place criteria. Tan delta--1 and storage modulus-1 are measured as described herein at a frequency of between 0.01 and 1 Hz at 37 degrees C. It is believed that 37 degrees C more closely mimics body temperature as well as the conditions under which the adhesive will perform.

For the adhesives of the present disclosure, the adhesive may be configured such that the adhesive exhibits a tan delta--1 value and a storage modulus-1 value which allows the adhesive to attach to the macro surface structure of outer cover fabrics. Adhesives of the present disclosure may exhibit a tan delta--1 value of between about 0.10 to about 0.40, between 0.10 to about 0.30, or between 0.14 to 0.30, when measured over a frequency range of between 0.01Hz and 1 Hz at 37 degrees C in accordance with the Frequency Sweep - Oscillatory Rheometry Test Method described herein, specifically reciting all values within this range and any ranges created therein or thereby. Adhesives of the present disclosure may exhibit a storage modulus-1 value of between about 10 kPa and about 50 kPa, between about 15 kPa and about 48 kPa, or between about 20 and about 45 kPa, specifically reciting all values within these ranges and any ranges created therein or thereby, according to the Frequency Sweep - Oscillatory Rheometry Test Method disclosed herein. It is worth noting that the adhesives of the present disclosure may exhibit the above tan delta--1 value over the entire range of 0.01 Hz to 1 Hz. So, at 0.01 Hz and at 1 Hz, the tan delta--1 value may be between 0.01 to 0.40, specifically reciting all values within this range and any ranges created therein or thereby.

During the application and subsequent initial wear of an absorbent insert in the outer cover, the adhesive can have a long time to be able to flow and create intimate contact with the fibers of the outer cover. However, within the frequency range specified, there is no fixed time in which the absorbent insert is applied to the outer cover - quite the opposite in fact. The application of the absorbent insert to the outer cover, from a time perspective, is highly variable. And as noted, initial application is critical in establishing good adhesion between the adhesive and the outer cover material.

The tan delta-1 and storage modulus-1 (collectively the "dampening factor") of the adhesive may be important for determining the behavior of the adhesive regarding initial attachment and staying in place. However, the inventors have also found that for removal of the absorbent insert from the outer cover, the damping factor at a higher frequency may also be important, since the consumer typically removes the product in a fraction of a second. Assuming the absorbent insert can be removed in 0.01s or more, for removal, a tan delta--2 value and storage modulus-2 value can be measured between 50 and 100 Hz at 37 degrees C. As a competing interest to the desirable attributes of an adhesive in the description of tan delta--1, the adhesives of the present disclosure may delaminate adhesively from the outer cover and have high enough cohesive strength to minimize the likelihood of residue on the outer cover post insert removal. Adhesive residue left over from absorbent insert removal may create a negative image in the mind of the user of the absorbent article brand, and may also inhibit future adhesion of inserts to the outer cover.

Adhesives of the present disclosure may exhibit a tan delta--2 value of 2.0 or less, 1.9 or less or 1.75 or less, over a frequency range of between 50 and 100 Hz at 37 degrees C, when measured in accordance with the Frequency Sweep - Oscillatory Rheometry Test Method disclosed herein, specifically reciting all values within the range and any ranges created thereby. For example, these adhesives of the present disclosure may exhibit a tan delta--2 value of between about 0.5 and about 2.0, between about 0.7 and about 1.9, or between about 1.0 to about 1.75, at a frequency of between 50 and 100 Hz at 37 degrees C, specifically reciting all values within these ranges and any ranges created therein or thereby. Adhesives of the present disclosure may have a storage modulus-2 value of between about 50 kPa and about 150 kPa, between about 60 kPa and about 125 kPa, or between about 65 kPa and about 110 kPa in accordance with the Frequency Sweep - Oscillatory Rheometry Test Method described herein specifically reciting all values within these ranges and any ranges created therein or thereby. It is worth noting that the adhesives of the present disclosure exhibit the above tan delta--2 and storage modulus-2 values over the entire range of 50 Hz to 100 Hz. So, at 50 Hz and at 100 Hz, the tan delta--2 value may be 2.0 or less, 1.9 or less or 1.75 or less, specifically reciting all values within this range and any ranges created thereby.

The inventors have also found that an adhesive of the present disclosure that exhibits little to no residue left on an outer cover upon removal of an insert may also have a surface energy value of greater than 26 mJ/m$^2$, greater than 30 mJ/m$^2$, greater than 35 mJ/m$^2$, greater than 40 mJ/m$^2$, greater than 42 mJ/m$^2$, between about 26 mJ/m$^2$ and about 75 mJ/m$^2$, between about 30 mJ/m$^2$ and about 70 mJ/m$^2$, between about 35 mJ/m$^2$ and about 65 mJ/m$^2$, between about 40 mJ/m$^2$ and about 60 mJ/m$^2$, or between about 42 mJ/m$^2$ and about 58 mJ/m$^2$, specifically reciting all values within these ranges and all ranges created therein or thereby, according to the Surface Energy Test Method disclosed herein. Additionally, the inventors have found that the adhesives of the present disclosure may exhibit a surface polarity that is a percentage of total surface energy of greater than 0.5%, greater than 1.0%, between about 0.5% and about 10%, between about 0.5% and about 8%, between about 1.0% and about 6%, or between about 1.0% and about 4%, specifically reciting all values within these ranges and all ranges created therein or thereby, according to the Surface Energy Method disclosed herein.

Regarding debonding (i.e., removal of the insert from the outer cover), the inventors have also found that the yield stress of the adhesive can provide additional information regarding whether the adhesive will leave residue on the undergarment during removal. The adhesives of the present disclosure can exhibit a yield stress of 25 kPa or greater, 30 kPa or greater, or 35 kPa or greater, or between about 35 kPa and about 95 kPa, between about 30 kPa and about 80 kPa, or between about 35 kPa and about 70 kPa, specifically reciting all values within these ranges and any ranges created thereby, when measured at 37 degrees C, in accordance with the Extensional Test Method for Yield Stress described herein.

Without wishing to be bound by theory, it is believed that an adhesive undergoes stress during removal. While in use, the adhesive creates an adhesive interface between the backsheet of the absorbent insert and the adhesive as well as an adhesive interface between the adhesive and the outer cover. It is believed that where the yield stress of the adhesive is below about 25 kPa, the adhesive interfaces survive removal and instead, the adhesive fails cohesively. This cohesive failure can lead to increased residue left on the outer cover. However, where the adhesive yield stress is above about 25 kPa, it is believed that the adhesive has sufficient strength to cause the adhesive to break the adhesive interface between the adhesive and the outer cover, thereby reducing the likelihood of adhesive residue being left behind on the outer cover.

In various embodiments, the engagement region may be disposed proximate to a lateral end of the absorbent insert 31, 33. For example, the absorbent insert may comprise a fastening component 1101 or receiving component 112I having an outboard-most edge 210 as shown in Fig. 5, for example. The outboard lateral edge may be at least partially coterminous with a lateral end as shown in Fig. 5. As shown in Fig. 6, the outboard lateral edge 210 may be separated from a lateral end by a maximum distance, D, of about 10 mm or less, or about 5 mm or less, or from about 1 mm to about 10 mm, reciting for said range every 1 mm increment therein. One or both of the longitudinal edges may be at least partially coterminous with the longitudinal edges of the insert 36, 37 as shown in Fig. 5. Alternatively, one or both of the longitudinal edges 214, 216 may be disposed inboard of the respective longitudinal edge(s) of the insert.

When attached (i.e., the engaged configuration 300), the absorbent article 10 comprises an insert-outer cover peel strength measured in N/cm according to the Insert-Outer Cover Peel Strength Test Method described herein. The insert-outer cover peel strength may be at least 0.02 N/cm, from about 0.02 N/cm to about 0.8 N/cm, from about 0.05 N/cm to about 0.75 N/cm or from about 0.05 N/cm to about 0.5 N/cm, specifically reciting every 0.01 N/cm increments within the specified ranges and all ranges formed therein or thereby.

It is also believed that repeated insert-outer cover peel strength testing using the same outer cover may assist in detecting residue, or build-up of adhesive, left on the outer cover. Residue left on the outer cover may reduce the effectiveness of the attachment means, either making it too weak or too strong. It is contemplated that performing repeated Insert-Outer Cover Peel Strength Tests may assist in detecting instances where adhesive residue is left on an outer cover after removal of an insert. In instances where residue remains on an outer cover, repeated Insert-Outer Cover Peel Strength Tests may show in increase or decrease in peel strength over repeated testing. On the other hand, where little to no residue is left on an outer cover, repeated Insert-Outer Cover Peel Strength Tests may show generally the same peel strength results from test to test.

Within the engagement region 200, the external layer 86 of the backsheet and the inner layer 85 of the backsheet are attached, having backsheet layer peel strength measured in N/cm according to the Peel Strength Test Method. As noted above, the inner layer may comprise a film and the external layer may comprise a nonwoven; in which case, the backsheet layer peel strength is also referred to as the backsheet film-to-nonwoven peel strength. The backsheet layer peel strength in the engagement region may be at least 0.85 N/cm, at least 0.95 N/cm, at least 1.1 N/cm, between about 0.85 N/cm and about 10 N/cm, between about 0.95 N/cm and about 8 N/cm, or between about 1.1 N/cm and about 6.5 N/cm, specifically reciting every 0.01 N/cm increments within the specified ranges and all ranges formed therein or thereby. The backsheet layers may be attached by a second bonding 204, which may be any suitable means, including but not limited to adhesive (such as hotmelt adhesive), mechanical bonding, heat bonding, pressure bonding and combinations thereof. In nonlimiting examples, the second bonding comprises adhesive. The bonding may be continuous or discontinuous. As noted above, where the bonding comprises adhesive bonding may be provided in patterns like slots, spirals, or the like. The backsheet layer peel strength is greater than the insert-outer cover peel strength. The backsheet layer peel strength is at least about 20% greater, or at least about 40% greater, or at least about 50% greater, or from about 10% to about 200% greater, or from about 20% to about 150% greater than the insert-outer cover peel strength, reciting for each range each 5% increment therein. In this way, when the insert is disengaged from the outer cover, the backsheet will remain intact due to the bond strength. This is especially beneficial when the engagement region is disposed near a lateral edge of the absorbent insert where there is little to no area that is initially free from attachment and capable of reducing the peel force necessary to detach the outer cover from the absorbent insert. The difference in peel strength may be effectuated by various means including, for example, different types of bonds, bonding material including bonding material combinations, relative basis weight of adhesive or other attachment means, bond frequency, bond patterns, bond area and combinations thereof. The first bonding may differ from the second bonding by at least one of the group consisting of: bonding material, continuous or discontinuous bonding, bond patterns, bond area, bond frequency.

Additionally, or alternatively, in the engagement region, the backsheet may be joined to the absorbent core, having a backsheet-to-core peel strength measured in N/cm according to the Peel Strength Test Method herein. More particularly, the backsheet may be attached to a substrate 49 of the absorbent core. Even more particularly, the backsheet inner layer 85 may be attached to the absorbent core substrate 49. The backsheet-to-core peel strength in the engagement region may be at least 0.85 N/cm, at least 0.95 N/cm, at least 1.1 N/cm, between about 0.85 N/cm and about 12 N/cm, between about 0.95 N/cm and about 10 N/cm, between about 1.1 N/cm and about 8 N/cm, or between about 1.25 N/cm and about 7.5 N/cm, specifically reciting every 0.01 N/cm increments within the specified ranges and all ranges formed therein or thereby. The backsheet is attached to the core by a third bonding 206, which may be any suitable means, including but not limited to adhesive (such as hotmelt adhesive), mechanical bonding, heat bonding, pressure bonding and combinations thereof. In nonlimiting examples, the third bonding comprises adhesive. The bonding may be continuous or discontinuous. As noted above, where the bonding comprises adhesive bonding may be provided in patterns like slots, spirals, or the like. In various embodiments, the backsheet-to-core peel strength is greater than the insert-outer cover peel strength. The backsheet-to-core peel strength may be at least about 10% greater, or at least about 20% greater, or at least about 30% greater, or from about 10% to about 100% greater, or from about 20% to about 80% greater than the insert-outer cover peel strength, reciting for each range each 5% increment therein. In this way, when the insert is disengaged from the outer cover, the layers of the absorbent insert, in particular the backsheet, will remain intact due to the bond strength between the backsheet and core. The difference in peel strength may be effectuated by various means including, for example, different types of bonds, bonding material including bonding material combinations, relative basis weight of adhesive or other attachment means, bond frequency, bond patterns, bond area and combinations thereof. The first bonding may differ from the third bonding by at least one of the group consisting of: bonding material, continuous or discontinuous bonding, bond patterns, bond area, bond frequency.

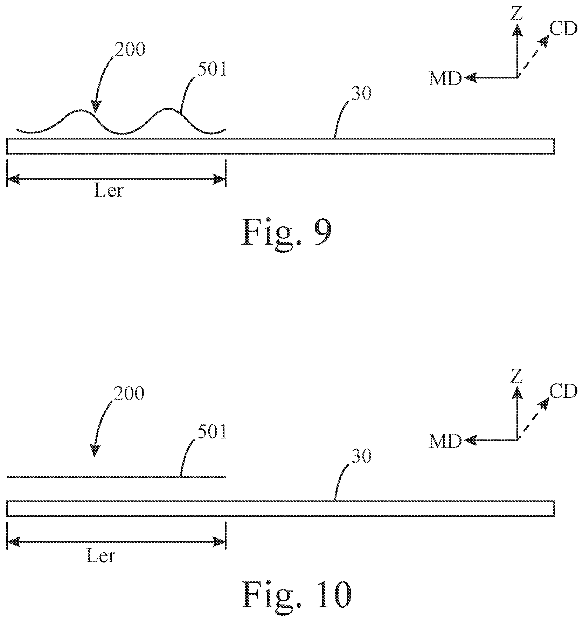

Where the first bonding 202 (bonding of the outer cover to the absorbent insert) is formed by means of an adhesive, the engagement region 200 of the absorbent insert containing the adhesive may be in direct or indirect contact with a release paper 501 prior to bonding the absorbent insert to the outer cover (i.e., the absorbent insert and the outer cover are in an unengaged configuration). The release paper may prevent unintended engagement of the adhesive with surfaces other than the engagement region 200 of the outer cover. The inventors have found that certain arrangements of the release paper in relation to the absorbent insert and/or the backsheet of the absorbent insert may result in improved performance of the adhesive in bonding the absorbent insert to the outer cover. For example, as shown in Fig. 9, when the release paper 501 is disposed over the engagement region 200 such that the release paper does not lay relatively flat against the engagement region 200 (the release paper exhibits some elevation in the z-direction away from the engagement region), the inventors have found that adhesive used as the bonding means is likely to be removed from the insert upon removal of the release paper 501. On the other hand, when the release paper 501 is disposed flush against the engagement region 200, thus exhibiting little to no elevation in the z-direction away from the engagement region 200, as shown in Fig. 10, adhesive used as a bonding means is likely to remain on the engagement region 200 of the absorbent insert 30 upon removal of the release paper 501. Without wishing to be bound by theory, it is believed that at least some of the properties of the adhesive that allow it to successfully bond the absorbent insert with the outer cover while also allowing the absorbent insert to be removed from the outer cover with little to no adhesive residue remaining may also cause the adhesive to adhere to the release paper when it pulls away in the z-direction from the engagement region shortly after application.

The inventors have found that when the length of the engagement region of the absorbent insert 30 that is overlapped by release paper 501 when the release paper 501 is bonded to the absorbent insert 30 (Ler) is similar to the length of the release paper in a flat-out configuration (Lrp), the adhesive is more likely to remain on the engagement region 200 of the absorbent insert 30 upon removal of the release paper 501. In other words, when the release paper is disposed in a relatively flat configuration - meaning little to no elevation away from the absorbent insert in the z-direction - over the engagement region when bonded to the absorbent insert, the inventors have found that removal of the release paper is less likely to remove significant amounts of adhesive from the engagement region of the absorbent insert. This may allow the absorbent insert to better bond with the outer cover and remain bonded with the outer cover until separation of the absorbent insert from the outer cover by a user.

A ratio of the length of the engagement region of the absorbent insert 30 that is overlapped by release paper 501 when the release paper 501 is bonded to the absorbent insert 30 (Ler) to the length of the release paper in a flat-out configuration (Lrp) may be between about 0.9:1 and about 1:1, between about 0.93:1 and about 1:1, between about 0.95:1 and about 1:1, or between about 0.97:1 and about 1:1.

Bio-Sourced Materials

Components of the disposable absorbent article can at least partially be comprised of bio-sourced content as described in U.S. Pat. Pub. Nos. 2007/0219521 A1, 2011/0139658 A1, 2011/0139657 A1, 2011/0152812 A1, and 2011/0139659 A1. These components include, but are not limited to, topsheets, backsheet films, backsheet nonwovens, side panels, leg gasketing systems, superabsorbent, acquisition layers, core wrap materials, adhesives, outer covers, fastener systems, and landing zones. In at least one embodiment, a disposable absorbent article component comprises a bio-based content value from about 10% to about 100%, or from about 25% to about 75%, or from about 50% to about 60% using ASTM D6866-10, method B. In order to apply the methodology of ASTM D6866-10 to determine the bio-based content of any component, a representative sample of the component must be obtained for testing. In at least one embodiment, the disposable absorbent article component can be ground into particulates less than about 20 mesh using known grinding methods (e.g., WILEY® mill), and a representative sample of suitable mass taken from the randomly mixed particles.

Examples

Comparative Example 1 and Example 1 are tested for rheological and mechanical properties discussed herein. The results of the rheological and mechanical property analysis are shown in TABLE 1.

Comparative Example 1 is a commercially available styrene block copolymer-based adhesive. Comparative Example 1 is the same adhesive formulation as Sample 7 in commonly assigned U.S. Patent App. No. 17/237174.

Example 1 is a styrene block copolymer-based adhesive.

TABLE 1:

|  | Comparative Example 1 | Example 1 |
|---|---|---|
| Tan Delta-1 | 0.11 - 0.44 | 0.14 - 0.20 |
| Tan Delta-2 | 1.89 - 2.32 | 1.41 - 1.98 |
| Storage Modulus-1 | 17 kPa - 29 kPa | 25kPa - 38kPa |
| Storage Modulus-2 | 89 kPa- 125kPa | 75 kPa- 92kPa |

(continued)

|  | Comparative Example 1 | Example 1 |
|---|---|---|
| Yield Stress | 16 kPa | 35 kPa |
| Surface Energy | 41 mJ/m$^2$ | 47 mJ/m$^2$ |
| Polar Component of Surface Energy | 0.03 % | 1.4 % |

As shown in TABLE 1, Example 1 exhibits a generally lower Tan Delta-2 as compared to Comparative Example 1, indicating that Example 1 may have more robust cohesive properties upon removal of an insert from an outer cover, therefore leaving less residue behind on the outer cover to interfere with future insert attachments. In addition, and as discussed above, the yield stress score of Example 1 further indicates the adhesive is likely to leave less residue behind upon removal from an outer cover. The surface energy and polar component of surface energy scores of Example 1 are further indications that the adhesive Example 1 is less likely to leave residue upon removal from an outer cover. The Tan Delta-1 score and Storage Modulus-1 sore indicate that Example 1 is likely to provide robust adhesion to attach an insert to an outer cover.

The adhesives of Comparative Example 1 and Example 1 are applied to the backsheets of inserts and applied to outer covers, according to the Insert-Outer Cover Peel Strength Test Method described herein, to test peel strength and to identify any residue after removal of the insert from the outer cover.

Example 2 utilizes the adhesive composition of Example 1, applied to a portion of the backsheet of an insert at a rate of 10 gsm.

Example 3 utilizes the adhesive composition of Example 1, applied to a portion of the backsheet of an insert at a rate of 12 gsm.

Example 4 utilizes the adhesive composition of Example 1, applied to a portion of the backsheet of an insert at a rate of 15 gsm.

Comparative Example 2 utilizes the adhesive composition of Comparative Example 1, applied to a portion of the backsheet of an insert at a rate of 15 gsm.

TABLE 2:

|  | Peel Strength (N/cm) |
|---|---|
| Example 2 | 0.14 |
| Example 3 | 0.15 |
| Example 4 | 0.15 |
| Comparative Example 2 | 0.78 |
| Backsheet Layer Peel Force | 1.37 |
| Backsheet-Core Peel Force | 1.51 |

The examples found in TABLE 2 are measured for peel strength according to the Insert-Outer Cover Peel Strength Test Method described herein. As shown in TABLE 2, Examples 2-4, which all utilize the adhesive formulation of Example 1, exhibit lower peel strength as compared to Comparative Example 2. In addition, Backsheet Layer Peel Force and Backsheet-Core Peel force are tested on the insert, and are significantly higher as compared to the insert-outer cover peel strength of Examples 2-4, indicating that the integrity of the insert will likely be maintained upon removal of the insert from the outer cover. Examples 2-4 also demonstrate no visible residue left on the outer cover upon removal of the insert upon visual inspection.

TEST METHODS:

*Caliper Test Method*

The caliper, or thickness, of a test specimen is measured as the distance between a reference platform on which the specimen rests and a pressure foot that exerts a specified amount of pressure onto the specimen over a specified amount of time. All measurements are performed in a laboratory maintained at 23 °C $\pm$ 2 C° and 50% $\pm$ 2% relative humidity and test specimens are conditioned in this environment for at least 2 hours prior to testing.

Caliper is measured with a manually operated micrometer equipped with a pressure foot capable of exerting a steady pressure of 0.50 kPa $\pm$ 0.01 kPa onto the test specimen. The manually operated micrometer is a dead-weight type instrument with readings accurate to 0.01 mm. A suitable instrument is Mitutoyo Series 543 ID-C Digimatic, available from VWR International, or equivalent. The pressure foot is a flat ground circular movable face with a diameter that is smaller than the test specimen and capable of exerting the required pressure. A suitable pressure foot has a diameter of 25.4 mm; however, a smaller or larger foot can be used depending on the size of the specimen being measured. The test specimen is supported by a horizontal flat reference platform that is larger than and parallel to the surface of the pressure foot. The system is calibrated and operated per the manufacturer's instructions.

Obtain a test specimen by removing it from an absorbent article, if necessary. When excising the test specimen from an absorbent article, use care to not impart any contamination or distortion to the test specimen layer during the process. The test specimen is obtained from an area free of folds or wrinkles, and it must be larger than the pressure foot.

To measure caliper, first zero the micrometer against the horizontal flat reference platform. Place the test specimen on the platform with the test location centered below the pressure foot. Gently lower the pressure foot with a descent rate of 3.0 mm $\pm$ 1.0 mm per second until the full pressure is exerted onto the test specimen. Wait 5 seconds and then record the caliper of the test specimen to the nearest 0.001 mm. In like fashion, repeat for a total of ten replicate test specimens. Calculate the arithmetic mean for all caliper measurements and report as Caliper to the nearest 0.001 mm.

*Insert-Outer Cover Peel Strength Test Method*

This Insert-Outer Cover Peel Strength Test Method is used to determine the force required to peel a strip of outer cover material (for example, a wearer-facing portion of an outer cover) from the attachment means on a garment-facing side of an absorbent insert. Peel force is measured on a constant rate of extension tensile tester interfaced to a computer (a suitable tensile tester interfaced with a computer such as MTS model Alliance RT/1 with TestWorks 4$^®$ software or equivalent is used). The tensile tester is in a temperature-controlled room at 22°C $\pm$ 2°C and 50 $\pm$ 10% relative humidity. The instrument is calibrated according to the manufacturer's instructions. The data acquisition rate is set to at least 50 Hertz. The grips used for the test are wider than the sample. The grips are air actuated grips designed to concentrate the entire gripping force along a single line perpendicular to the direction of testing stress having one flat surface and an opposing face from which protrudes a half round (radius = 6 mm, e.g., part number: 56-163-827 from MTS Systems Corp.) or equivalent grips, to minimize slippage of the sample. The load cell is selected so that the forces measured are between 10% and 90% of the capacity of the load cell used.

The initial distance between the lines of gripping force (gauge length) is set at 25.4 mm. The load reading on the instrument is zeroed to account for the mass of the fixture and grips. A padded weight assembly is used to ensure adequate and even attachment of the microfiber material strip to the attachment means, where applicable. The weight assembly must impart a pressure of 26 - 27 g/cm$^2$ with a base that has about the same length and width as that of the test sample (width determined at widest location on test sample). The weight assembly is constructed as follows. Lay a single layer of polyethylene film (0.02 - 0.04 mm thick; any convenient source) flat on a bench surface. A piece of flexible insulation foam (Buna-N/PVC, 1 inch thick, density of 4.5 pounds/cubic foot; available from McMaster-Carr, Princeton, NJ, or equivalent) that is cut to the predetermined base size is laid centered on top of the film. A metal weight (same length and width as the predetermined base size) with a handle is then attached to the insulation foam using double sided tape. Next the polyethylene film is wrapped around the insulation foam and secured to the sides of the metal weight using transparent tape.

A strip of material that forms a portion of the inner layer of the outer cover is used. Prepare a strip of outer cover material that has a width equal to the widest portion of any joining pattern (bond pattern or adhesive pattern, for example) with a length that is about 30 mm longer than twice the longitudinal length of the joining pattern on the sample. The outer cover material strip must be sufficiently wide enough to cover the entire width of the joining pattern and long enough to cover the entire length of the joining pattern, overlap itself entirely and still have enough excess leftover to insert into the upper grip of the tensile tester. The length of the material strip is cut in a direction that is perpendicular to the waistband and should not include any seams, waistband material or gusset. Note which side of the material is intended to face the body (i.e., the inside of the outer cover). The length of the outer cover material strip must be long enough to cover the entire longitudinal length of the joining pattern on the test sample. A fresh outer cover material strip is used for each test sample. Remove the insert test sample from the remainder of the article by cutting through the article along the longitudinal edges of the insert sample, longitudinally extending 125 mm inboard from the longitudinally most outboard edge with the sample width of 25 mm. Keep the release paper attached. Care should be taken to prevent damage of the specimen during the separation process.

Attach the insert test sample to the outer cover material strip as follows. Remove the release paper from the insert sample, placed on flat surface with wearer-facing surface down. Laterally center the outer cover material strip over the insert sample (wearer-facing surface of outer cover facing the insert sample). Position the leading edge of the outer cover material strip at a distance no more than 1 cm above the front edge of the insert test sample. Ensure the longitudinal axis of the outer cover material strip is aligned with the longitudinal axis of the insert sample. Continue to apply the outer cover material strip over the remaining portion of the attachment pattern without creating any wrinkles in the outer cover or insert sample. By design, there will be an excess length of outer cover material (or standard cotton if used to supplement the length of the outer cover material) trailing off the back longitudinal end of the sample, referred to as the trailing end of the outer cover material strip. Center and then place the prepared weight assembly over the sample on top of the attached outer cover material strip. After 30 ± 2 seconds have elapsed, remove the weight assembly, and set it aside. The sample is now prepped for testing and must be analyzed within 1 minute after the weight assembly has been removed.

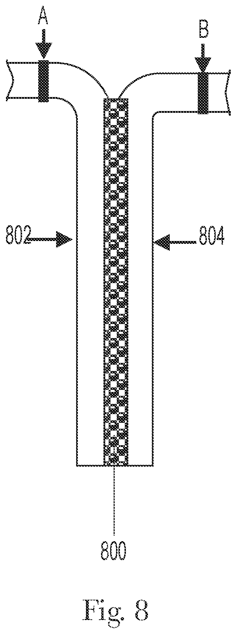

Referring to Fig. 8, for correct segment mounting into the test apparatus a minimum of 25 mm of unbonded material edge is required, for both substrates of the composite. If one, or both substrates, have less than 25 mm of unbonded material edge available, a section of tape such as 3M SCOTCH® 234, or similar, folded to form a flap can be adhered to the exposed material edge until the minimum 25 mm length is obtained. If no unbonded material edge is available, the composite substrates can be carefully separated using tweezers until a minimum of 5 mm unbonded edge is available.

The segment is mounted into the grips in a manner such that there is no slack and the load measured is between 0.00 N and 0.02 N. The segment is mounted in the center of the grips, such that the segment peeling direction is parallel to the applied tensile stress. The segment is placed between the grips such that the longitudinal dimension of the bonding region will be perpendicular to the grip apexes, where the first grip is holding the first substrate 802 at Grip Line A and the second grip is holding the second substrate 804 at Grip Line B, thereby peeling the first substrate from the second substrate in a 180° peeling direction. The peel test is initiated, and the segment is extended at 305 mm/min, with a data acquisition rate of at least 50 Hertz, until the substrates separate completely. The peel displacement of the segment is reported on the x axis in millimeters of crosshead travel, while the separation force of the segment is reported on the y axis in Force (Newtons (N)). The "peel strength" for each specimen is the maximum force measured for each sample during delamination reported to the nearest 0.01N and divided by the width of the sample, in cm (2.54cm in the sample collected as directed). The result is reported as peel strength in N/cm to the nearest 0.01N/cm

*Peel Strength Test Method* (Absorbent insert backsheet film-to-nonwoven or backsheet-to-core)

The "Peel Strength Test Method measures the peak amount of tensile force per unit of width required to pull two joined portions of an absorbent insert apart during a 180° peel test using a tensile strength apparatus.

A suitable tensile tester interfaced with a computer such as MTS model Alliance RT/1 with TestWorks 4® software or equivalent is used. The tensile tester is located in a temperature-controlled room at 22°C ± 2°C and 50 ± 10% relative humidity. The instrument is calibrated according to the manufacturer's instructions. The data acquisition rate is set to at least 50 Hertz. The grips used for the test are wider than the sample. The grips are air actuated grips designed to concentrate the entire gripping force along a single line perpendicular to the direction of testing stress having one flat surface and an opposing face from which protrudes a half round (radius = 6 mm, e.g., part number: 56-163-827 from MTS Systems Corp.) or equivalent grips, to minimize slippage of the sample. The load cell is selected so that the forces measured are between 10% and 90% of the capacity of the load cell used.

The initial distance between the lines of gripping force (gauge length) is set at 25.4 mm. The load reading on the instrument is zeroed to account for the mass of the fixture and grips.

Locate the composite of interest (backsheet film-to-nonwoven or backsheet-to-core). If the composite includes portions extending beyond the area of overlap, remove said portions along the longitudinal edge of the overlapping area.

Remove the composite from the remainder of the article by cutting through the article along the longitudinal edges of the composite, longitudinally extending 125 mm inboard from the longitudinally most outboard edge with the sample width of 25 mm while maintaining attachment between the components forming the composite. Care should be taken to prevent damage of the specimen during the separation process.

Referring to Fig. 8, for correct segment mounting into the test apparatus a minimum of 25 mm of unbonded material edge is required, for both substrates of the composite. If one, or both substrates, have less than 25 mm of unbonded material edge available, a section of tape such as 3M SCOTCH® 234, or similar, folded to form a flap can be adhered to the exposed material edge until the minimum 25 mm length is obtained. If no unbonded material edge is available, the composite substrates can be carefully separated using tweezers until a minimum of 5 mm unbonded edge is available.

The segment is mounted into the grips in a manner such that there is no slack and the load measured is between 0.00 N and 0.02 N. The segment is mounted in the center of the grips, such that the segment peeling direction is parallel to the applied tensile stress. The segment is placed between the grips such that the longitudinal dimension of the bonding region will be perpendicular to the grip apexes, where the first grip is holding the first substrate 802 at Grip Line A and the second grip is holding the second substrate 804 at Grip Line B, thereby peeling the first substrate from the second substrate in a 180° peeling direction. The peel test is initiated, and the segment is extended at 305 mm/min, with a data acquisition rate of at least 50 Hertz, until the substrates separate completely. The peel displacement of the segment is reported on the x axis in millimeters of crosshead travel, while the separation force of the segment is reported on the y axis in Force (Newtons (N)). The "peel strength" for each specimen is the maximum force measured for each sample during delamination reported to the nearest 0.01N and divided by the width of the sample, in cm (2.54cm in the sample collected as directed). The final result is reported as peel strength in N/cm to the nearest 0.01N/cm

*Surface Energy Test Method*

Contact angles on substrates are determined using a goniometer and appropriate image analysis software (a suitable instrument is the DSA100 Drop Shape Analyzer, Kruss GmbH, Hamburg, Germany, or equivalent) fitted with a gas tight syringe and steel needle capable of dispensing 1.0 uL drops. Two test fluids are used: Type II reagent water (distilled) in accordance with ASTM Specification D1193-99 and 99+% purity diiodomethane (both available from Sigma Aldrich, St. Louis, MO). Contact angles from these two test fluids can further be used to calculate surface energy based on the Fowkes Theory. All testing is to be performed at about 23 °C ± 2 C° and a relative humidity of about 50% ± 2%.

Contact angle measurements are made on test specimens taken from the raw material or obtained from a material layer removed from an absorbent article. When excising the material layer from an absorbent article, use care to not impart any contamination or distortion to the layer during the process. When the test location is on an adhesive, do not remove any protective cover that may be present until the measurement is to be made in order to decrease the likelihood of contaminating the adhesive layer. The test specimen must be of an appropriate size such that it fits onto the stage of the goniometer and can accommodate any potential spreading of the applied test fluid. Prepare enough test specimens for a total of twenty test locations (ten locations for each test fluid).

Set up the goniometer on a vibration-isolation table and level the stage according to the manufacturer's instructions. The video capture device must have an acquisition speed capable of capturing at least 10-20 images from the time the drop of test fluid hits the surface of the specimen to the time it cannot be resolved from the specimen's surface. A capture rate of 900 images/sec is typical. Depending on the hydrophobicity / hydrophilicity of the specimen, the drop may or may not rapidly wet the surface of the test specimen. If the test specimen is capable of absorbing the test fluid at a slow rate, the images should be acquired until no more than 2% of the volume of the drop is absorbed into the specimen. If the test specimen is capable of absorbing the test fluid quickly, the first resolved image should be used if the second image shows more than 2% volume loss.

Place the specimen on the goniometer's stage and adjust the hypodermic needle to the distance from the surface recommended by the instrument's manufacturer (typically 3 mm). If necessary, adjust the position of the specimen to place the test location under the needle tip. Focus the video device such that a sharp image of the drop on the surface of the specimen can be captured. Start the image acquisition. Deposit a 1.0 $\mu$L ± 0.05 $\mu$L drop of diiodomethane onto the specimen. If there is visible distortion of the drop shape due to movement, repeat at a different, but equivalent, test location. Make two angle measurements on the drop (one on each drop edge) from the image at which there is no more than a 2% loss in drop volume. If the contact angles on two edges are different by more than 4°, the values should be excluded, and the test repeated at an equivalent location on the specimen. Identify nine additional equivalent sites on the specimen and repeat for a total of 10 measurements (20 angles). Calculate the arithmetic mean for all replicates and report as Contact Angle with Diiodomethane to the nearest 0.01°. In like fashion, measure the contact angle with water as the test fluid, and report as Contact Angle with Water to the nearest 0.01°.

To calculate surface energy, the contact angle for both diiodomethane and water must be tested as described above. The value of contact angle for each test fluid is then used, along with additional information about each test fluid, to make calculations using the Young-Dupre equation and the Fowkes Theory as follows:

The Young-Dupre equation (Eq 1)

$$W_{sl} = \gamma_l (\cos\theta + 1)$$

The Fowkes Theory (Eq 2)

$$W_{sl} = 2[(\gamma_l^D * \gamma_s^D)^{0.5} + (\gamma_l^P * \gamma_s^P)^{0.5}]$$

Combining Eq 1 and Eq 2 to get Eq 3

$$\frac{\gamma_l(\cos\theta + 1)}{2} = (\gamma_l^D * \gamma_s^D)^{0.5} + (\gamma_l^P * \gamma_s^P)^{0.5}$$

where:
$W_{sl}$ = work of adhesion;

θ = the average contact angle for the respective test fluid on the test specimen;
$\gamma_l$ and $\gamma_s$ = the surface tension of the test liquid and test specimen, respectively, in mJ/m$^2$ ;
$\gamma^D$ and $\gamma^P$ = the dispersive and polar components of the surface tension, respectively, in mJ/m$^2$;
and the properties of the test fluids are as follows:

|  | Surface Tension ($\gamma_l$) (mJ/m$^2$) | | |
| --- | --- | --- | --- |
| Solvent | Dispersive ( $\gamma_l^D$ ) | Polar ( $\gamma_l^P$ ) | Total ($\gamma_l$) |
| Diiodomethane | 50.8 | 0.0 | 50.8 |
| Water | 26.4 | 46.4 | 72.8 |

Eq 3 can be further simplified when a purely dispersive solvent such as diiodomethane is used since the polar component is zero and $\gamma_l^D = \gamma_l$ , as follows:
Eq 4 (Eq 3 simplified for a purely dispersive solvent)

$$\gamma_s^D = \frac{\gamma_l(\cos\theta + 1)^2}{4}$$

Using the surface tension values from the table and θ (measured) for diiodomethane, Eq 4 can be solved for the dispersive component of surface energy ($\gamma^D_s$) and reported to the nearest 0.01 mJ/m$^2$. Now using the values from the table and θ (measured) for water, along with the previously calculated value for $\gamma^D_s$, Eq 3 can be solved for the polar component of surface energy ($\gamma^P_s$) and reported to the nearest 0.01 mJ/m$^2$. Total surface energy of the test specimen ($\gamma_s$) is calculated as the sum of $\gamma^D_s + \gamma^P_s$ and reported to the nearest 0.01 mJ/m$^2$. Now calculate surface polarity by dividing the polar component of surface energy ($\gamma^P_s$) by the total surface energy ($\gamma_s$), then multiplying by 100 and reporting to the nearest 0.01%.

*Frequency Sweep - Oscillatory Rheometry Test Method*

The Oscillatory Rheometry Test Method is used to measure the Storage Modulus and the Damping Factor (also known as tan delta and storage modulus) of adhesive tapes or hot melt adhesive compositions. A rotational rheometer (such as DHR 3, TA Instruments, New Castle, DE, USA, or equivalent) capable of sample temperature control with a precision equal to or exceeding 0.5 degrees C over at least the range of 0 degrees C to 150 degrees C. The rheometer needs to have a normal force control system to enable the axial force control on the specimen with an accuracy of 0.1 N. The rheometer is operated in a parallel plate configuration with 25 mm stainless steel parallel-plate tooling for hot melt adhesives and 20 mm for adhesive tapes or 8 mm when the adhesive tape width is smaller than 20mm.

Adhesive tapes specimen preparation:

For adhesive tapes individual specimens for measurement are punched with a circular sample cutter of 20 mm diameter or 8 mm if the adhesive tape width is smaller than 20 mm. The release films are removed, and the measurement specimen is placed centered on the lower plate of the rheometer at 23 +/- 0.5 degrees C. The upper rheometer plate is lowered until it gets in contact with the adhesive tape. The upper plate is pressed with approximately 5 - 10 N axial force at 23 +/- 0.5 degrees C for about 30 - 90 sec onto the adhesive tape to ensure full contact. After that the axial force control is set to 1.0 N and be maintained within ± 0.1 N of force during the experiment.

Hot melt specimen preparation:

The rheometer is heated to 150 degrees C, the adhesive or polymer composition is introduced in the rheometer, the gap is set to 1050 μm, excess protruding sample is trimmed, and the gap is then set to 1000 μm. (The axial force control of the rheometer is set to 0 N and be maintained within ± 0.1 N of axial force during the experiment, thereby thermal expansion/contraction of the sample itself is compensated by adjusting the gap in order to avoid overfilling or underfilling in addition to the abovementioned compensation of the tooling.) The rheometer is then allowed to cool to 37 degrees C.

Measurement:

The sample is equilibrated at 37 degrees C +/- 0.5 degrees C for 300 s. An oscillatory frequency sweep with 0.1% strain is performed from 0.01 - 100 Hz [1/s] at 37 degrees C +/- 0.5 degrees C. In a logarithmic matter 10 points per decade are acquired (see frequency table).

Analysis:

From the frequency sweep, the storage modulus G' and the tan delta is calculated and recorded from 0.01 to 100 Hz for 10 frequencies per decade in a logarithmic matter. The storage modulus values are reported in kilopascal (KPa) to the nearest 0.01 KPa. The tan delta and storage modulus are recorded to the nearest hundredth.

*Extensional Test Method for Yield Stress*

The Extensional Test Method is used to determine the Yield Stress for a specimen of an adhesive composition. A thin film specimen formed of adhesive composition is analyzed with a rotational rheometer fitted with a specialized fixture with counter rotating rollers, and the stress associated with extensional strain imparted is measured and recorded.

Instrumental setup:

A rotational rheometer (ARES G2, TA Instruments, New Castle, DE, USA, or equivalent) is fitted with a fixture that has counter rotating cylindrical metal rollers specifically designed for the interrogation of extension deformation of films. An example of a suitable fixture is the Extensional Viscosity Fixture, or EVF (EVF, TA Instruments, or equivalent). The rheometer is further fitted with a forced-convection oven FCO (FCO, TA Instruments, or equivalent) and cooling system (ACS 2, TA Instruments, or equivalent) capable of controlling temperate from at least -50 to 250 °C to a within a tolerance of 0.5 °C.

Specimen preparation:

If the adhesive is mounted on a substrate the adhesive needs to be separated before preparing the adhesive film as test specimen. This can be done by extracting the adhesive with an organic solvent like Tetrahydrofuran (THF) and evaporating the entire solvent afterwards gently at room temperature 20°C - 30°C to obtain the adhesive for test specimen preparation.

To prepare the test specimen 2 g to 10g of the adhesive composition is placed in a circular polytetrafluoroethane (PTFE) bowl with a flat bottom (diameter of 60 mm or 25 mm$\pm$ 2 mm) and introduced into a vacuum oven held at 170°C. After 15 minutes at ambient pressure, the pressure is lowered to 10 mbar, and the adhesive composition is subsequently held at 170°C and at 10 mbar for 45 minutes to remove air bubbles from the adhesive composition. If 170°C is insufficient to melt the adhesive composition s a temperature $30 \pm 10$ °C above the melting temperature of the polymer material composition is used. The adhesive composition is removed from the vacuum oven and allowed to cool to ambient lab conditions ($23 \pm 2$ °C) for $90 \pm 30$ minutes, at which point the adhesive composition is removed from the PTFE bowl and placed between 2 sheets of siliconised paper (such as product number 114918, Mondi Group, Hilm, Austria, or equivalent). A metal shim $500 \pm 30$ $\mu$m in thickness is used in the heated press as a spacer to obtain a film thickness of 500 $\mu$m when pressed with a heated press at 90 °C for 60 seconds at a pressure sufficient to form a polymeric film. If 90 °C is insufficient to press a uniform flat film, a temperature approximately $10 \pm 5$ °C below the melting point of the sample material composition such that the sample material composition is in a semi-solid state is used. The film is stored at least 120 hours in the laboratory at $23 \pm 2$ °C prior to testing. From the film individual specimens for measurement are punched with a sample cutter to the final specimen dimensions of 20.0 mm by 10.0 mm by 500 $\mu$m.

Measurement:

To secure the specimen film to the EVF, the specimen is briefly pressed onto the cylinders of the EVF to secure it to the cylinder surfaces. The specimen is placed with its length perpendicular to the axis of rotation of the cylinders.

The specimen mounted on the EVF is then placed in the forced convection oven of the rheometer for thermal conditioning and is kept isothermal at $37 \pm 0.5$ °C for $300 \pm 10$ s. After this time has elapsed, the specimen is mechanically conditioned. To mechanically condition the specimen, the torque transducer is zeroed, and the sample is put under a pre-stretch rate of 0.001 s$^{-1}$ for 0.30 s and then allowed to relax for 60 s (in this method, all strain is expressed in terms of Hencky strain, also known as "true strain" or "logarithmic strain.").

The measurement is performed in the FCO oven at 37 °C $\pm$ 0.5 °C. The strain rate extension for the measurement

is 1 s$^{-1}$, and the strain at maximum extension is 4.0. After measurement, the specimen is checked for rupturing. If it has ruptured, the location of the break is noted. If the rupture is approximately in the middle between the two cylinders of the EVF, the data collected are deemed acceptable. Otherwise, if the polymeric film break is at or close to the rotating cylinders, the results are discarded, and the measurement performed again on a replicate specimen.

Analysis:

For the extensional stress calculation, a constant volume is assumed. From the raw torque versus angular displacement data recorded by the rheometer, extensional stress (in kilopascals, or kPa) versus Hencky strain data are calculated. The data are plotted in semilogarithmic fashion with Hencky strain on the abscissa (linear scale) and extensional stress on the ordinate (logarithmic scale). A linear range is sought in this plot between a Hencky strain of 0.7 and 1.3. The value of the fitted line at a Hencky strain of zero (that is, the y-intercept), is defined as the Yield Stress, which is reported in kPa to the nearest kilopascal.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. An absorbent article (10) comprising:

   an absorbent insert (30) comprising a topsheet (32), a backsheet (34), and an absorbent core (44) disposed between the topsheet and backsheet; the backsheet comprising a nonwoven (86) joined to a film (85) and a backsheet film-to-nonwoven peel strength measured in N/cm in an engagement region (200);
   an outer cover (20);
   a fastening system (100) capable of joining the absorbent insert to the outer cover; wherein:

   in an engaged configuration, the article comprises an insert-outer cover peel strength measured in N/cm in the engagement region; and
   the backsheet film-to-nonwoven peel strength is greater than the insert-outer cover peel strength;
   wherein the backsheet film-to-nonwoven peel strength is at least 20% greater than the insert-outer cover.

2. The absorbent article according to claim 1 wherein the nonwoven and film are joined by a second bonding (204), and wherein the fastening system comprises a first bonding (202), wherein the first bonding differs from the second bonding by at least one of the group consisting of: bonding material, continuous or discontinuous bonding, bond patterns, bond area, bond frequency.

3. The absorbent article according to any one of the preceding claims wherein the nonwoven is joined to the film by adhesive.

4. The absorbent article according to any one of the preceding claims wherein the fastening system comprises adhesive and/or a hook-and-loop system.

5. The absorbent article according to claim 4, wherein the fastening system comprises adhesive, and wherein the adhesive has a yield stress of greater than 25 kPa, according to the Extensional Test Method for Yield Stress.

6. The absorbent article according to claim 4, wherein the fastening system comprises adhesive, and wherein the adhesive has a surface energy greater than 42 mJ/m$^2$, according to the Surface Energy Test Method.

7. The absorbent article according to claims 4 or 6 wherein the fastening system comprises adhesive, and wherein the adhesive has a polar component of surface energy greater than 0.5%, according to the Surface Energy Test Method.

8. The absorbent article according to any one of the preceding claims wherein the backsheet film-to-nonwoven peel strength is at least 0.85 N/cm.

9. The absorbent article according to any one of the preceding claims wherein an insert-outer cover peel strength is at least 0.02 N/cm.

10. The absorbent article according to any one of the preceding claims wherein the absorbent insert is disposable.

11. The absorbent article according to any one of the preceding claims wherein the absorbent insert comprises an absorbent capacity of at least 200 g.

12. The absorbent article according to any one of the preceding claims wherein the absorbent core is substantially free of cellulosic material.

13. The absorbent article according to any one of the preceding claims wherein the engagement region is at least partially disposed within 5 mm of a first lateral end of the absorbent insert as measured in the longitudinal direction.

14. The absorbent article according to any one of the preceding claims wherein:

the absorbent core comprises a substrate (49);
the backsheet is joined to the substrate in the engagement region, and the absorbent insert comprises a backsheet-to-core substrate peel strength measured in N/cm; and
the backsheet-to-core peel strength is greater than the insert-outer cover peel strength;

15. The absorbent article according to claim 14 wherein the backsheet-to-core peel strength is at least 20% greater than the insert-outer cover peel strength.

16. The absorbent article according to claims 14 or 15 wherein the substrate comprises a nonwoven.

17. The absorbent article according to any one of claims 14-16 wherein the substrate is joined to the backsheet by adhesive.

18. The absorbent article according to any one of claims 14-17 wherein the fastening system comprises a first bonding and the substrate and backsheet are joined by a third bonding (206), and wherein the first bonding differs from the third bonding by at least one of the group consisting of: bonding material, continuous or discontinuous bonding, bond patterns, bond area, bond frequency.

19. The absorbent article according to any one of claims 14-18 wherein the backsheet-to-core peel strength is at least 0.85 N/cm.

## Patentansprüche

1. Absorptionsartikel (10), umfassend:

einen Absorptionseinsatz (30), umfassend eine Oberschicht (32), eine Unterschicht (34) und einen Absorptionskern (44), der zwischen der Oberschicht und der Unterschicht angeordnet ist; die Unterschicht umfassend ein Vlies (86), das an eine Folie (85) gebunden ist, und eine Folie-zu-Vlies-Schälfestigkeit der Unterschicht, die in N/cm in einem Eingriffsbereich (200) gemessen wird;
einen Außenmantel (20);
ein Befestigungssystem (100), das in der Lage ist, den Absorptionseinsatz an den Außenmantel zu binden; wobei: in einer Eingriffskonfiguration, der Artikel eine Einsatz-Außenmantel-Schälfestigkeit umfasst, die in N/cm in dem Eingriffsbereich gemessen wird; und
die Folie-zu-Vlies-Schälfestigkeit der Unterschicht größer als die Einsatz-Außenmantel-Schälfestigkeit ist; wobei die Folie-zu-Vlies-Schälfestigkeit der Unterschicht wenigstens 20 % größer als der Einsatz-Außenmantel ist.

2. Absorptionsartikel nach Anspruch 1, wobei das Vlies und die Folie durch eine zweite Verbindung (204) gebunden sind und wobei das Befestigungssystem eine erste Verbindung (202) umfasst, wobei sich die erste Verbindung von der zweiten Verbindung durch wenigstens eines aus der Gruppe unterscheidet, bestehend aus: Verbindungsmaterial, ununterbrochene oder unterbrochene Verbindung, Verbindungsmuster, Verbindungsfläche, Verbindungsfrequenz.

3. Absorptionsartikel nach einem der vorstehenden Ansprüche, wobei das Vlies durch Haftmittel an die Folie gebunden ist.

4. Absorptionsartikel nach einem der vorstehenden Ansprüche, wobei das Befestigungssystem Haftmittel und/oder ein Klettverschlusssystem umfasst.

5. Absorptionsartikel nach Anspruch 4, wobei das Befestigungssystem Haftmittel umfasst und wobei das Haftmittel eine Fließspannung von größer als 25 kPa nach dem Extensionalprüfverfahren für Fließspannung aufweist.

6. Absorptionsartikel nach Anspruch 4, wobei das Befestigungssystem Haftmittel umfasst und wobei das Haftmittel eine Oberflächenenergie aufweist, die größer als 42 mJ/m$^2$ nach dem Oberflächenenergieprüfverfahren ist.

7. Absorptionsartikel nach Anspruch 4 oder 6, wobei das Befestigungssystem Haftmittel umfasst und wobei das Haftmittel einen polaren Bestandteil der Oberflächenenergie aufweist, der größer als 0,5 % nach dem Oberflächenenergieprüfverfahren ist.

8. Absorptionsartikel nach einem der vorstehenden Ansprüche, wobei die Folie-zu-Vlies-Schälfestigkeit der Unterschicht wenigstens 0,85 N/cm beträgt.

9. Absorptionsartikel nach einem der vorstehenden Ansprüche, wobei eine Einsatz-Außenmantel-Schälfestigkeit wenigstens 0,02 N/cm beträgt.

10. Absorptionsartikel nach einem der vorstehenden Ansprüche, wobei der Absorptionseinsatz wegwerfbar ist.

11. Absorptionsartikel nach einem der vorstehenden Ansprüche, wobei der Absorptionseinsatz ein Absorptionsvermögen von wenigstens 200 g umfasst.

12. Absorptionsartikel nach einem der vorstehenden Ansprüche, wobei der Absorptionskern im Wesentlichen frei von cellulosischem Material ist.

13. Absorptionsartikel nach einem der vorstehenden Ansprüche, wobei der Eingriffsbereich wenigstens teilweise innerhalb von 5 mm eines ersten seitlichen Endes des Absorptionseinsatzes, gemessen in der Längsrichtung, angeordnet ist.

14. Absorptionsartikel nach einem der vorstehenden Ansprüche, wobei:

   der Absorptionskern ein Trägermaterial (49) umfasst;
   die Unterschicht an das Trägermaterial in dem Eingriffsbereich gebunden ist und der Absorptionseinsatz eine Unterschicht-zu-Kernträgermaterial-Schälfestigkeit gemessen in N/cm umfasst; und
   die Unterschicht-zu-Kern-Schälfestigkeit größer als die Einsatz-Außenmantel-Schälfestigkeit ist;

15. Absorptionsartikel nach Anspruch 14, wobei die Unterschicht-zu-Kern-Schälfestigkeit wenigstens 20 % größer als die Einsatz-Außenmantel-Schälfestigkeit ist.

16. Absorptionsartikel nach Anspruch 14 oder 15, wobei das Trägermaterial ein Vlies umfasst.

17. Absorptionsartikel nach einem der Ansprüche 14 bis 16, wobei das Trägermaterial durch Haftmittel an die Unterschicht gebunden ist.

18. Absorptionsartikel nach einem der Ansprüche 14 bis 17, wobei das Befestigungssystem eine erste Verbindung umfasst und das Trägermaterial und die Unterschicht durch eine dritte Verbindung (206) gebunden sind und wobei sich die erste Verbindung von der dritten Verbindung durch wenigstens eines aus der Gruppe unterscheidet, bestehend aus: Verbindungsmaterial, ununterbrochene oder unterbrochene Verbindung, Verbindungsmuster, Verbindungsfläche, Verbindungsfrequenz.

19. Absorptionsartikel nach einem der Ansprüche 14 bis 18, wobei die Unterschicht-zu-Kern-Schälfestigkeit wenigstens 0,85 N/cm beträgt.

**Revendications**

1. Article absorbant (10) comprenant :

   un insert absorbant (30) comprenant une feuille de dessus (32), une feuille de fond (34), et une âme absorbante (44) disposée entre la feuille de dessus et la feuille de fond ; la feuille de fond comprenant un non-tissé (86) relié à un film (85) et une résistance au pelage film/non-tissé de feuille de fond mesurée en N/cm dans une région de mise en prise (200) ;
   une couverture externe (20) ;
   un système de fixation (100) capable de relier l'insert absorbant à la couverture externe ; dans lequel : dans une configuration en prise, l'article comprend une résistance au pelage insert/couverture externe mesurée en N/cm dans la région de prise ; et
   la résistance au pelage film/non-tissé de feuille de fond est supérieure à la résistance au pelage insert/couverture externe ;
   dans lequel la résistance au pelage film/non-tissé de feuille de fond est au moins 20 % supérieure à celle insert/couverture externe.

2. Article absorbant selon la revendication 1, dans lequel le non-tissé et le film sont reliés par une deuxième liaison (204), et dans lequel le système de fixation comprend une première liaison (202), dans lequel la première liaison diffère de la deuxième liaison par au moins l'un parmi le groupe constitué de : matériau de liaison, liaison continue ou discontinue, motifs de liaison, zone de liaison, fréquence de liaison.

3. Article absorbant selon l'une quelconque des revendications précédentes, dans lequel le non-tissé est relié au film par adhésif.

4. Article absorbant selon l'une quelconque des revendications précédentes, dans lequel le système de fixation comprend de l'adhésif et/ou un système crochet et boucle.

5. Article absorbant selon la revendication 4, dans lequel le système de fixation comprend de l'adhésif, et dans lequel l'adhésif a une limite d'élasticité supérieure à 25 kPa, selon la méthode d'essai d'extension pour la limite d'élasticité.

6. Article absorbant selon la revendication 4, dans lequel le système de fixation comprend de l'adhésif, et dans lequel l'adhésif a une énergie de surface supérieure à 42 mJ/m$^2$, selon la méthode d'essai d'énergie de surface.

7. Article absorbant selon les revendications 4 ou 6, dans lequel le système de fixation comprend de l'adhésif, et dans lequel l'adhésif a une composante polaire d'énergie de surface supérieure à 0,5 %, selon la méthode d'essai d'énergie de surface.

8. Article absorbant selon l'une quelconque des revendications précédentes, dans lequel la résistance au pelage film/non-tissé de feuille de fond est d'au moins 0,85 N/cm.

9. Article absorbant selon l'une quelconque des revendications précédentes, dans lequel une résistance au pelage insert/couverture externe est d'au moins 0,02 N/cm.

10. Article absorbant selon l'une quelconque des revendications précédentes, dans lequel l'insert absorbant est jetable.

11. Article absorbant selon l'une quelconque des revendications précédentes, dans lequel l'insert absorbant comprend une capacité d'absorption d'au moins 200 g.

12. Article absorbant selon l'une quelconque des revendications précédentes, dans lequel l'âme absorbante est sensiblement exempte de matériau cellulosique.

13. Article absorbant selon l'une quelconque des revendications précédentes, dans lequel la région de mise en prise est au moins partiellement disposée dans les 5 mm d'une première extrémité latérale de l'insert absorbant telle que mesurée dans la direction longitudinale.

14. Article absorbant selon l'une quelconque des revendications précédentes, dans lequel :

l'âme absorbante comprend un substrat (49) ;

la feuille de fond est reliée au substrat dans la région de mise en prise, et l'insert absorbant comprend une résistance au pelage feuille de fond/âme mesurée en N/cm ; et

la résistance au pelage feuille de fond/âme est supérieure à la résistance au pelage insert/couverture externe ;

15. Article absorbant selon la revendication 14, dans lequel la résistance au pelage feuille de fond/âme est au moins 20 % supérieure à la résistance au pelage insert/couverture externe.

16. Article absorbant selon les revendications 14 ou 15, dans lequel le substrat comprend un non-tissé.

17. Article absorbant selon l'une quelconque des revendications 14 à 16, dans lequel le substrat est relié à la feuille de fond par adhésif.

18. Article absorbant selon l'une quelconque des revendications 14 à 17, dans lequel le système de fixation comprend une première liaison et le substrat et la feuille de fond sont reliés par une troisième liaison (206), et dans lequel la première liaison diffère de la troisième liaison par au moins l'un dans le groupe constitué de : matériau de liaison, liaison continue ou discontinue, motifs de liaison, zone de liaison, fréquence de liaison.

19. Article absorbant selon l'une quelconque des revendications 14 à 18, dans lequel la résistance au pelage feuille de fond/âme est d'au moins 0,85 N/cm.

Fig. 1

Fig. 2

Fig. 3

Fig. 4

Fig. 5

Fig. 6

Fig. 7

Fig. 8

Fig. 9

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014138274 A1
- US 6120487 A
- US 8998870 B
- US 9089456 B
- US 8435223 B
- US 9011402 B
- US 8808263 B
- US 8759605 B
- US 8932273 B
- US 9078789 B
- US 9387138 B
- US 687493 A
- US 12687412 B
- US 12687528 B
- US 12687425 B, Roe
- US 7223818 B
- US 7211531 B
- US 7060149 B
- US 6964720 B
- US 6905987 B
- US 6890872 B
- US 6884494 B
- US 6878647 B
- US 5518801 A
- US 20080319407 A
- US 20080045917 A
- US 20070293111 A
- US 20070287983 A
- US 20070287348 A
- US 20070249254 A
- US 20070203301 A
- US 20050164587 A
- US 533472 A
- US 15074675 B
- US 62855001 B
- US 3848594 A
- US 4662875 A
- US 4846815 A
- US 4894060 A
- US 4946527 A
- US 5151092 A
- US 6432098 B
- US 684230 A
- US 16545425 B
- US 63028021 A
- US 8546641 B
- US 5628097 A, Benson
- US 20160136014, Arora
- US 7744576 B
- US 20110268932 A1
- US 20110319848 A1
- US 20110250413 A1
- US 9072634 B
- US 491642 A
- US 15232901 B
- WO 200059430 A
- WO 9510996 A
- US 5700254 A
- WO 02067809 A
- US 8939957 B
- US 237174 A
- US 20070219521 A1
- US 20110139658 A1
- US 20110139657 A1
- US 20110152812 A1
- US 20110139659 A1